# EUROPEAN PATENT APPLICATION

(11) **EP 3 712 243 A1**
(43) Date of publication of application: **23.09.2020**
(21) Application number: 20163740.2
(22) Date of filing: 17.03.2020
(51) Int. Cl.: C12M 1/26

(54) **LIQUID SET FOR DROPLET DISCHARGING APPARATUS**

(30) Priority: 20.03.2019 JP 2019053550; 27.06.2019 JP 2019120412
(71) Applicant: Ricoh Company, Ltd., Tokyo 143-8555 (JP); The University of Tokyo, Bunkyo-ku, Tokyo 113-8654 (JP)
(72) Inventor: SHIONOIRI, Momoko, Tokyo 143-8555 (JP); YAGINUMA, Hidekazu, Tokyo 143-8555 (JP); KUBO, Chihiro, Tokyo 143-8555 (JP); SAKAI, Takamasa, Tokyo 113-8654 (JP)
(74) Representative: J A Kemp LLP

(57) **Abstract**

Provided is a liquid set for a droplet discharging apparatus, the liquid set including: Liquid A containing a multi-branched polymer A, the polymer containing, as a backbone, a polyethylene glycol containing at least three branches, the branches containing one or more electrophilic functional groups in at least one of a side chain(s) and an end(s); and Liquid B containing a multi-branched polymer B, the polymer containing, as a backbone, a polyethylene glycol containing at least three branches, the branches containing one or more nucleophilic functional groups in at least one of a side chain(s) and an end(s); the Liquid A and the Liquid B each having a pH of from 5 to 10, and containing the multi-branched polymer at a concentration of from 0.3% by mass to 20% by mass.

## Description

### BACKGROUND OF THE INVENTION

### Field of the Invention

The present invention relates to a liquid set for a droplet discharging apparatus, which liquid set enables precise three-dimensional arrangement of cells.

### Description of the Related Art

Owing to the recent development of stem cell technologies, techniques for artificially forming a three-dimensional structure containing a plurality of cells have been developed. Known examples of methods for arranging the cells for the preparation of the three-dimensional structure include the cell sheet method, the spheroid layering method, the gel extrusion method, and the ink jet method.

The cell sheet method is a method in which thin monolayer sheets having a thickness of less than 0.01 µm are prepared, and the sheets are layered on each other to prepare a three-dimensional structure. However, the cell sheet method does not enable efficient large-scale production of a three-dimensional structure since the cell sheets to be layered are prepared one by one.

The spheroid layering method is a method in which spheroids (cell aggregates) are layered to prepare a three-dimensional structure. The gel extrusion method is a method in which a gel containing cells is continuously extruded from a nozzle to layer the cells, to prepare a three-dimensional structure. However, the spheroid layering method and the gel extrusion method only allow arrangement of cells in units of not less than several hundred micrometers, and therefore do not allow precise three-dimensional arrangement of cells. Regarding the gel extrusion method, there is a concern that a considerable degree of pressure may be applied to the cells.

On the other hand, a droplet discharging apparatus based on the ink jet method allows formation of a three-dimensional structure in which cells are precisely three-dimensionally arranged. However, the viscosity of the ink for the apparatus needs to be low enough to allow discharge of the ink by the ink jet head as a droplet discharging head. Moreover, for the precise three-dimensional arrangement of the cells, the shape-forming time needs to be short so as to allow survival of the cells in the gel.

For example, Patent Documents 1, 2, and 3 disclose methods in which an aqueous sodium alginate solution is discharged by ink jetting to produce a gel having a three-dimensional structure in which cells are precisely three-dimensionally arranged. By selecting the raw material of the aqueous sodium alginate solution, the viscosity can be reduced, and the gelation time can be shortened, so that precise three-dimensional arrangement is possible. However, since the gelation occurs by ion cross-linking between the alginate and chloride, immersion of the gel in a buffer for cells or in a culture medium leads to gradual elimination of the chloride in the gel, resulting in degradation of the gel and difficulty in maintenance of the shape. Moreover, it is known that cells included in the alginate gel cannot survive for a long period in the gel. One possible solution is degradation of the gel with EDTA or the like. However, this degradation instantly causes degradation of the gel, which destroys the arrangement of the cells, and which may affect the cells.

For example, Patent Documents 4, 5, and 6 disclose methods in which a liquid containing gelatin and fibrinogen is discharged by ink jetting to produce a gel having a three-dimensional structure. In cases where the concentrations of the gelatin and the fibrinogen are adjusted to low concentrations, the liquid can be discharged by ink jetting, and cells included in the gel can survive for a long period in the gel. However, in the cases where the concentrations of the gelatin and the fibrinogen are adjusted to low concentrations, gelation becomes impossible, or the gelation time increases, resulting in precipitation of the cells under their own weight after their three-dimensional arrangement. Thus, arbitrary arrangement of the cells cannot be easily achieved.

### Related Art Documents

### [Patent Documents]

[Patent Document 1] Japanese Patent No. 4974144
[Patent Document 2] Japanese Unexamined Patent Application Publication No. 2017-163931
[Patent Document 3] Japanese Unexamined Patent Application Publication No. 2017-169560
[Patent Document 4] Japanese Unexamined Patent Application Publication No. 2017-209103
[Patent Document 5] Japanese Unexamined Patent Application Publication No. 2008-17798
[Patent Document 6] Japanese Patent No. 5540304

In view of the conventional circumstances described above, an object of the present invention is to provide a liquid set for a droplet discharging apparatus, which liquid set allows survival of cells, formation of a three-dimensional structure, and maintenance of the shape of the three-dimensional structure obtained.

### SUMMARY OF THE INVENTION

For solving the above problems, the liquid set for a droplet discharging apparatus according to the present invention includes: Liquid A containing a multi-branched polymer A, the polymer containing, as a backbone, a polyethylene glycol containing at least three branches, the branches containing one or more electrophilic functional groups in at least one of a side chain(s) and an end(s); and Liquid B containing a multi-branched polymer B, the polymer containing, as a backbone, a polyethylene glycol containing at least three branches, the branches containing one or more nucleophilic functional groups in at least one of a side chain(s) and an end(s); the Liquid A and the Liquid B each having a pH of from 5 to 10, and containing the multi-branched polymer at a concentration of from 0.3% by mass to 20% by mass.

By the present invention, a liquid set for a droplet discharging apparatus, which liquid set allows survival of cells, formation of a three-dimensional structure, and maintenance of the shape of the three-dimensional structure obtained, can be provided.

### BRIEF DESCRIPTION OF THE DRAWINGS

FIG. 1 is a schematic diagram illustrating an example of an electromagnetic-valve-type discharging head;
FIG. 2 is a schematic diagram illustrating an example of a piezo-type discharging head;
FIG. 3 is a schematic diagram illustrating an example of modification of the piezo-type discharging head in FIG. 2;
FIG. 4 is a schematic diagram illustrating an example of a voltage applied to a piezoelectric element;
FIG. 5 is a schematic diagram illustrating another example of a voltage applied to a piezoelectric element;
FIG. 6 is a schematic diagram illustrating an ink droplet-observing mechanism;
FIG. 7 is an image of dead cells in Example 13 as viewed using 4D Viewer;
FIG. 8 is an image of all cells in Example 13 as viewed using 4D Viewer;
FIG. 9 is an image obtained by observation, from the upper side, of cells in a gel formed in Example 13; and
FIG. 10 is an image obtained by observation, from the upper side, of cells in a gel formed in Example 15.

### DESCRIPTION OF THE EMBODIMENTS

The present invention is described below in detail according to embodiments.

Since the embodiments described below are preferred embodiments of the present invention, various technically preferred limitations are given to the embodiments. However, as long as there is no description indicating limitation of the present invention in the following description, the scope of the present invention is not limited to these modes.

The liquid set for a droplet discharging apparatus according to the present invention includes: Liquid A containing a multi-branched polymer A, the polymer containing, as a backbone, a polyethylene glycol containing at least three branches, the branches containing one or more electrophilic functional groups in at least one of a side chain(s) and an end(s); and Liquid B containing a multi-branched polymer B, the polymer containing, as a backbone, a polyethylene glycol containing at least three branches, the branches containing one or more nucleophilic functional groups in at least one of a side chain(s) and an end(s). Each component is described below.

### <Multi-branched Polymer Containing Polyethylene Glycol as Backbone>

The multi-branched polymer used for the liquid set for a droplet discharging apparatus of the present invention, which polymer contains a polyethylene glycol as a backbone, is a polymer containing three or more polyethylene glycol branches, wherein molecules of the polymer cross-link to each other to form a network structure. In particular, four-branched polymers form homogeneous network structures, and gels having a four-branched polyethylene glycol backbone are generally known as Tetra-PEG gels. A Tetra-PEG has a network structure formed by cross-end coupling reaction between two kinds of four-branched polymers each containing an electrophilic functional group or a nucleophilic functional group in at least one of a side chain(s) and an end(s).

A past study has reported that a Tetra-PEG gel has an ideal homogeneous network structure (Matsunaga T, et al., Macromolecules, Vol.42, No.4, pp.1344-1351 (2009)). A Tetra-PEG gel can be simply prepared on site by mixing of two polymer liquids, and the gelation time can be controlled by adjusting the pH and the polymer concentration of each polymer liquid (which corresponds to each of Liquid A and Liquid B in the present invention). By discharging the Liquid A and the Liquid B through an ink jet head as a droplet discharging head, and then allowing gelation of the liquids to form a Tetra-PEG gel, a three-dimensional structure can be produced such that cells can be three-dimensionally arranged with the structure. Since the gel contains a polyethylene glycol as a major component, the gel has excellent biocompatibility.

The total number of the electrophilic functional group(s) in the polymer in Liquid A and the nucleophilic functional group(s) in the polymer in Liquid B is preferably not less than 6. Although these functional groups may be present in one or both of a side chain(s) and an end(s) of each polymer, the functional groups are preferably present in an end(s) of each polymer. The content of the electrophilic functional group in the polymer in Liquid A may be higher than the content of the nucleophilic functional group in the polymer in Liquid B in the composition. Alternatively, the content of the nucleophilic functional group in the polymer in Liquid B may be higher than the content of the electrophilic functional group in the polymer in Liquid A in the composition. In a preferred mode, two or more kinds of combination of Liquid A and Liquid B having different compositions may be used to once form two or more kinds of gel precursors having different compositions, and the gel precursors may be further cross-linked to obtain a gel having a three-dimensional structure.

The electrophilic functional group contained in the multi-branched polymer in Liquid A is preferably maleimidyl, which is an active ester group. When necessary, in addition to the maleimidyl, the polymer may contain N-hydroxy-succinimidyl (NHS), sulfosuccinimidyl, phthalimidyl, imidazoyl, acryloyl, nitrophenyl, or the like. Those skilled in the art can select and employ other known active ester groups as appropriate. Among the multi-branched polymer molecules contained in Liquid A, the composition of the electrophilic functional group may be either the same or different. The composition is preferably the same. In cases where the functional group composition is the same, reactivity with the nucleophilic functional group forming the cross-link is homogeneous, and therefore a gel having a homogeneous spatial structure can be easily obtained.

The nucleophilic functional group contained in the multi-branched polymer in Liquid B is preferably thiol. When necessary, in addition to the thiol, the polymer may contain amino, -CO₂PhNO₂ (wherein Ph represents o-, m-, or p-phenylene), or the like. Those skilled in the art can select and employ various nucleophilic functional groups as appropriate. Among the multi-branched polymer molecules contained in Liquid B, the composition of the nucleophilic functional group may be either the same or different. The composition is preferably the same. In cases where the functional group composition is the same, reactivity with the electrophilic functional group forming the cross-link is homogeneous, and therefore a gel having a homogeneous spatial structure can be easily obtained.

Preferred specific examples of a multi-branched polymer containing one or more maleimidyl groups in at least one of a side chain(s) and an end(s) and containing a polyethylene glycol as a backbone include, but are not limited to, compounds represented by the following Formula (I), which contains four polyethylene glycol backbone branches, and maleimidyl groups at the ends.

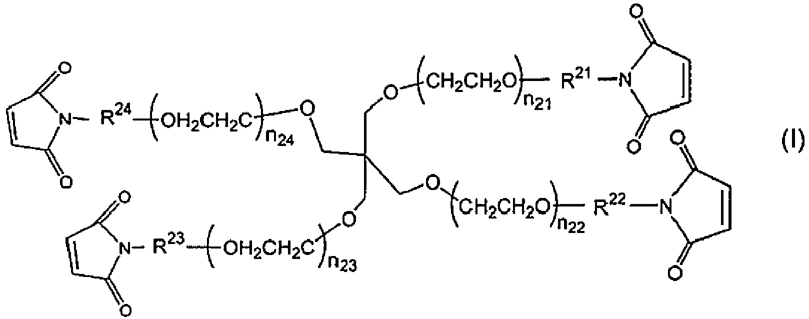

In the Formula (I), each of n₂₁ to n₂₄ may be either the same or different. As the values of n₂₁ to n₂₄ become close to each other, the gel can have a more homogeneous spatial structure, which is preferred because of a higher strength of the gel. n₂₁ to n₂₄ especially preferably have the same value. In cases where the values of n₂₁ to n₂₄ are too high, the gel has a lower strength, while in cases where the values of n₂₁ to n₂₄ are too low, the gel can be hardly formed due to steric hindrance of the compound. Thus, each of n₂₁ to n₂₄ appropriately has a value of from 5 to 300, preferably from 20 to 250, more preferably from 30 to 180, still more preferably from 45 to 115, especially preferably from 45 to 55. The multi-branched polymer in Liquid A has a weight average molecular weight of preferably from 5 × 10³ to 5 × 10⁴, more preferably from 7.5 × 10³ to 3 × 10⁴, still more preferably from 1 × 10⁴ to 2 × 10⁴.

In the Formula (I), R²¹ to R²⁴ are linker portions that link the functional groups to the core portion. Although R²¹ to R²⁴ may be either the same or different, R²¹ to R²⁴ are preferably the same from the viewpoint of producing a gel having a homogeneous spatial structure and a high strength. In Formula (I), R²¹ to R²⁴ are the same or different, and examples of R²¹ to R²⁴ include C₁-C₇ alkylene, C₂-C₇alkenylene, -NH-R²⁵-, -CO-R²⁵-, -R²⁶-O-R²⁷-, -R²⁶-NH-R²⁷-, -R²⁶-CO₂-R²⁷-, -R²⁶-CO₂-NH-R²⁷-, -R²⁶- CO-R²⁷-, -R²⁶-NH-CO-R²⁷-, and-R²⁶-CO-NH-R²⁷-. Here, R²⁵ represents C₁-C₇ alkylene; R²⁶ represents C₁-C₃ alkylene; and R²⁷ represents C₁-C₅ alkylene.

Preferred specific examples of a multi-branched polymer containing one or more thiol groups in at least one of a side chain(s) and an end(s) and containing a polyethylene glycol as a backbone include, but are not limited to, compounds represented by the following Formula (II), which contains four polyethylene glycol backbone branches, and thiol groups at the ends.

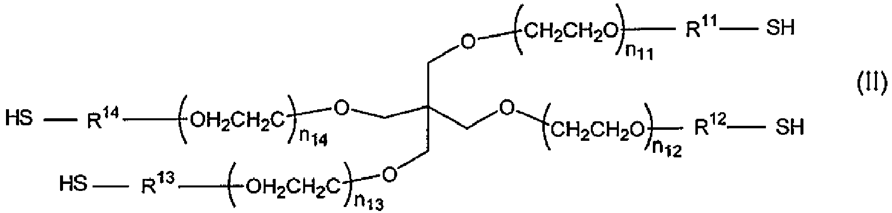

In the Formula (II), each of n₁₁ to n₁₄ may be either the same or different. As the values of n₁₁ to n₁₄ become close to each other, the gel can have a more homogeneous spatial structure, which is preferred because of a higher strength of the gel. n₁₁ to n₁₄ especially preferably have the same value. In cases where the values of n₁₁ to n₁₄ are too high, the gel has a lower strength, while in cases where the values of n₁₁ to n₁₄ are too low, the gel can be hardly formed due to steric hindrance of the compound. Thus, each of n₁₁ to n₁₄ has a value of preferably from 25 to 250, more preferably from 35 to 180, still more preferably from 50 to 115, especially preferably from 50 to 60. The multi-branched polymer in Liquid B has a weight average molecular weight of preferably from 5 × 10³ to 5 × 10⁴, more preferably from 7.5 × 10³ to 3 × 10⁴, still more preferably from 1 × 10⁴ to 2 × 10⁴.

In the Formula (II), R¹¹ to R¹⁴ are linker portions that link the functional groups to the core portion. Although R¹¹ to R¹⁴ may be either the same or different, R¹¹ to R¹⁴ are preferably the same from the viewpoint of producing a gel having a homogeneous spatial structure and a high strength. In Formula (II), R¹¹ to R¹⁴ are the same or different, and examples of R¹¹ to R¹⁴ include C₁-C₇ alkylene, C₂-C₇alkenylene, -NH-R¹⁵-, -CO-R¹⁵-, -R¹⁶-OR¹⁷-, -R¹⁶-NH-R¹⁷-, -R¹⁶-CO₂-R¹⁷-, -R¹⁶-CO₂-NH-R¹⁷-, -R¹⁶-CO-R¹⁷-, -R¹⁶-NH-CO-R¹⁷-, and-R¹⁶-CO-NH-R¹⁷-. Here, R¹⁵ represents C₁-C₇ alkylene; R¹⁶ represents C₁-C₃ alkylene; and R¹⁷ represents C₁-C₅ alkylene.

Here, "C₁-C₇ alkylene" means an alkylene group which may be branched and which has from 1 to 7 carbon atoms, and means a linear C₁-C₇ alkylene group, or a C₂-C₇ alkylene group having one or more branches (having from 2 to 7 carbon atoms including the carbon atoms in the branch(es)). Examples of the C₁-C₇ alkylene include methylene, ethylene, propylene, and butylene. More specific examples of the C₁-C₇ alkylene include -CH₂-,-(CH₂)₂-, -(CH₂)₃-, -CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

"C₂-C₇ alkenylene" means a linear or branched alkenylene group having from 2 to 7 carbon atoms, and containing one or more double bonds in the chain. Examples of the C₂-C₇ alkenylene include divalent groups containing a double bond, which groups are formed by elimination of from 2 to 5 hydrogen atoms from adjacent carbon atoms of the alkylene group.

In the present description, the alkylene group and the alkenylene group may contain one or more arbitrary substituents. Examples of such substituents include, but are not limited to, alkoxy, halogen atoms (any of a fluorine atom, chlorine atom, bromine atom, and iodine atom), amino, mono- or di-substituted amino, substituted silyl, acyl, and aryl. In cases where two or more substituents are contained, those substituents may be either the same or different.

As defined in the present description, in cases where a functional group "may have a substituent(s)", the type(s), the substitution site(s), and the number of the substituent(s) are not limited. In cases where two or more substituents are contained, the substituents may be either the same or different. Examples of the substituents include, but are not limited to, alkyl, alkoxy, hydroxy, carboxyl, halogen atoms, sulfo, amino, alkoxycarbonyl, and oxo. These substituents may further contain a substituent.

### <Type and Concentration of Buffer>

Each of Liquid A and Liquid B in the liquid set for a droplet discharging apparatus of the present invention preferably contains an appropriate buffer in addition to the multi-branched polymer component containing a polyethylene glycol as a backbone. Examples of the buffer include phosphate buffer, citrate buffer, citrate-phosphate buffer, acetate buffer, borate buffer, tartrate buffer, Tris buffer, Tris-HCl buffer, phosphate buffered saline, citrate-phosphate buffered saline, and cell culture media. The buffer in Liquid A and the buffer in Liquid B may be either the same or different. Each of the buffer in Liquid A and the buffer in Liquid B may be a mixture of two or more kinds of buffers.

In cases where the concentration of the buffer is too low, the pH buffering capacity in the solution is low, and therefore a gel having a high strength cannot be produced. On the other hand, in cases where the buffer concentration is too high, mixing of the multi-branched polymer component contained in Liquid A and containing a polyethylene glycol as a backbone, with the multi-branched polymer component contained in Liquid B and containing a polyethylene glycol as a backbone, is inhibited. Therefore, a gel having a high strength cannot be produced. Thus, the concentration of the buffer in each of Liquid A and Liquid B is preferably within the range of from 20 mM to 200 mM from the viewpoint of production of a gel having a homogeneous structure and a high strength.

### <pH of Buffer, and Concentration of Multi-branched Polymer Containing Polyethylene Glycol as Backbone>

As described above, the gelation time can be controlled by adjusting the pH of the buffer and the concentration of the multi-branched polymer which is contained in each of Liquid A and Liquid B and which contains a polyethylene glycol as a backbone. Thus, a gelation time optimal for three-dimensional arrangement of cells can be obtained by the adjustment. More specifically, a buffer is used such that the pH of each of Liquid A and Liquid B is adjusted to 5 to 10. The concentration of the multi-branched polymer which is contained in each of Liquid A and Liquid B and which contains a polyethylene glycol as a backbone is adjusted within the range of from 0.3% by mass to 20% by mass. The pH of each of Liquid A and Liquid B is preferably from 6 to 10, and the concentration of the multi-branched polymer which is contained in each of Liquid A and Liquid B and which contains a polyethylene glycol as a backbone is preferably from 1.7% by mass to 20% by mass.

In an acidic solution having a pH of less than 5 in which the concentration of the multi-branched polymer containing a polyethylene glycol as a backbone is less than 0.3% by mass, the nucleophilic functional group is likely to be in a cationic state, leading to repulsion from each other. As a result, reactivity between the nucleophilic functional group in the cationic state and the electrophilic functional group in the other polymer component decreases. Therefore, three-dimensional arrangement of cells is impossible. On the other hand, in cases where the concentration of the multi-branched polymer containing a polyethylene glycol as a backbone is higher than 20% by mass, the solution cannot be discharged by an ink jet head, and is therefore not suitable as an ink jet ink as a liquid for a droplet discharging apparatus. In an alkaline solution having a pH of more than 10, reactivity between the nucleophilic functional group and the electrophilic functional group is too high, so that the gelation time is abnormally short. Therefore, each polymer cannot be sufficiently dispersed throughout the gel, and the gel becomes fragile as a result. Thus, the solution is not suitable.

In cases where the pH of each of Liquid A and Liquid B is from 6 to 10, and the concentration, in each of Liquid A and Liquid B, of the multi-branched polymer containing a polyethylene glycol as a backbone is from 1.7% by mass to 20% by mass, the gel can be formed with better precision compared to alginate gel, which has been conventionally used. Thus, a more precise three-dimensional arrangement of cells is possible. Furthermore, in cases where cells capable of adhesive spreading such as fibroblasts are included in Liquid A and Liquid B, when the pH of each of Liquid A and Liquid B is set to 5 to 10, and the concentration of the multi-branched polymer containing a polyethylene glycol as a backbone is set to 0.3% by mass to 6.0% by mass, a sufficient space can be secured for allowing spreading of the cells embedded in the gel. Furthermore, by setting the concentration of the multi-branched polymer in each of Liquid A and Liquid B to 0.3% by mass to 4.0% by mass, the cells in the gel can be allowed to survive for a long period.

### <Viscosities of Liquid A and Liquid B>

In cases where the viscosity of each of Liquid A and Liquid B is too high, the liquid cannot be discharged by an ink jet head, so that the liquid is not suitable for a liquid set for a droplet discharging apparatus. More specifically, the viscosity of each of Liquid A and Liquid B at 25°C is set to not more than 30 mPa·s.

### <Molar Ratio between Nucleophilic Functional Group and Electrophilic Functional Group>

Liquid A and Liquid B are preferably mixed together such that the molar ratio between the nucleophilic functional group and the electrophilic functional group is within the range of from 0.5:1 to 1.5:1. Since the functional groups react with each other at 1:1 to form a cross-link, the closer the mixing molar ratio to 1:1, the more preferred. For obtaining a hydrogel having a high strength, the ratio is especially preferably within the range of from 0.8:1 to 1.2:1.

### < Self-Assembling Biomaterial>

One or both of Liquid A and Liquid B may contain a self-assembling biomaterial. The self-assembling biomaterial means a material derived from a living organism, which material can be formed into a tissue by mixing with another material, and/or adjusting the pH, temperature, and/or the like. The type and the like of the self-assembling biomaterial are not limited, and may be appropriately selected depending on the purpose. In particular, in cases where the self-assembling biomaterial contains a cell adhesion factor, adhesive spreading of cells can be allowed in a gel formed using the liquid set of the present invention. Examples of the self-assembling biomaterial include gellan gum, calcium alginate, agarose, guar gum, xanthan gum, carrageenan, pectin, locust bean gum, tamarind gum, diutan gum, carboxymethyl cellulose, polylactic acid, polyglycolic acid, collagen, gelatin, proteoglycan, hyaluronic acid, entactin, elastin, chitin, fibrinogen, cellulose, and Matrigel. Matrigel is a preparation of the soluble basement membrane extracted from murine sarcoma containing extracellular matrix protein. Matrigel contains, as major components, laminin and collagen, heparan sulfate proteoglycan, and the like. Any one of self-assembling biomaterials including those described above may be used individually, or two or more of such self-assembling biomaterials may be used in combination.

### <Cells>

One or both of Liquid A and Liquid B may contain suspended cells. The type and the like of the cells are not limited, and may be appropriately selected depending on the purpose. In terms of taxonomy, the cells may be, for example, eukaryotic cells, prokaryotic cells, multicellular organism cells, unicellular organism cells, or the like. Any cells may be used.

Examples of the eukaryotic cells include animal cells, insect cells, plant cells, and fungi. Any one type of these cells may be used individually, or two or more types of these cells may be used in combination. Among these, animal cells are preferred. In cases where the cells form a cell aggregate, the cells are more preferably adhesive cells having cell adhesiveness which is enough to allow adhesion of the cells to each other and to prevent isolation of the cells from each other as long as the cells are not subjected to a physicochemical treatment.

The adhesive cells are not limited, and may be appropriately selected depending on the purpose. Examples of the adhesive cells include differentiated cells and undifferentiated cells.

Examples of the differentiated cells include hepatocytes as parenchymal cells of the liver; stellate cells; Kupffer cells; vascular endothelial cells; endothelial cells such as sinusoidal endothelial cells and corneal endothelial cells; fibroblasts; osteoblasts; osteoclasts; periodontal membrane-derived cells; epidermal cells such as epidermal keratinocytes; tracheal epithelial cells; gastrointestinal epithelial cells; cervical epithelial cells; epithelial cells such as corneal epithelial cells; mammary cells; pericytes; muscle cells such as smooth muscle cells and cardiomyocytes; kidney cells; pancreatic Langerhans islet cells; nerve cells such as peripheral nerve cells and optic nerve cells; chondrocytes; and bone cells. The adhesive cells may be primary cells directly collected from a tissue or an organ, or may be subcultured cells obtained after several passages.

The undifferentiated cells are not limited, and may be appropriately selected depending on the purpose. Examples of the undifferentiated cells include pluripotent stem cells, such as embryonic stem cells, which are undifferentiated cells, and mesenchymal stem cells, which have pluripotency; unipotent stem cells such as vascular endothelial progenitor cells, which have unipotency; and iPS cells.

Examples of the prokaryotic cells include eubacteria and archaebacteria.

Specific examples of the cells include normal human skin fibroblasts. As the normal human skin fibroblasts, a commercially available product may be used. Examples of the commercially available product include CC2507 (trade name; manufactured by Lonza).

### <Other Components>

Liquid A and Liquid B may contain other components, if necessary. The other components are not limited, and may be appropriately selected depending on the purpose. Examples of the other components include culture media, cross-linking agents, pH adjusters, antiseptics, and antioxidants.

### <Culture Medium>

The culture medium is a solution containing components required for formation and maintenance of the three-dimensional structure. The medium prevents drying, and controls the external environment including the osmotic pressure. The culture medium is not limited, and may be appropriately selected from known culture media depending on the intended use. For a three-dimensional structure which does not need to be constantly immersed in a culture medium, such as skin whose surface is exposed to air, the culture medium may be removed as appropriate.

The culture medium is not limited, and may be appropriately selected depending on the purpose. Examples of the culture medium include various culture media classified according to the composition, such as natural media, semi-synthetic media, and synthetic media; and various culture media classified according to the shape, such as semi-solid media, liquid media, and powder media. Any one of these culture media may be used individually, or two or more types of these culture media may be used in combination. In cases where the cells are derived from an animal, any culture medium for use in animal cell culture may be used.

The culture medium for use in animal cell culture is not limited, and may be appropriately selected depending on the purpose. Examples of the culture medium include Dulbecco's Modified Eagle's Medium (D-MEM), Ham's F12 medium (Ham's Nutrient Mixture F12), D-MEM/F12 medium, McCoy's 5A medium, Eagle's MEM medium (Eagle's Minimum Essential Medium (EMEM)), αMEM medium (alpha Modified Eagle's Minimum Essential Medium; αMEM), MEM medium (Minimum Essential Medium), RPMI 1640 medium, Iscove's Modified Dulbecco's Medium (IMDM), MCDB131 medium, William's medium E, IPL 41 medium, Fischer's medium, StemPro 34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by StemCell Technologies Inc.), StemSpan SFEM (manufactured by StemCell Technologies Inc.), Stemline II (manufactured by Sigma-Aldrich), QBSF-60 (manufactured by Quality Biological, Inc.), StemPro hESC SFM (manufactured by Invitrogen), Essential 8 (registered trademark) medium (manufactured by Gibco), mTeSRlor 2 medium (manufactured by StemCell Technologies Inc.), Repro FF or Repro FF2 (manufactured by ReproCELL Inc.), PSGro hESC/iPSC medium (manufactured by System Biosciences, Inc.), NutriStem (registered trademark) medium (manufactured by Biological Industries), CSTI-7 medium (manufactured by Cell Science & Technology Institute, Inc.), MesenPRO RS medium (manufactured by Gibco), MF-Medium (registered trademark) mesenchymal stem cell growth medium (manufactured by Toyobo Co., Ltd.), Sf-900II (manufactured by Invitrogen), and Opti-Pro (manufactured by Invitrogen). Any one type of these culture media may be used individually, or two or more of these culture media may be used in combination.

The carbon dioxide concentration in the culture medium is not limited, and may be appropriately selected depending on the purpose. The carbon dioxide concentration is preferably from 2% to 5%, more preferably from 3% to 4%. In cases where the carbon dioxide concentration is from 2% to 5%, the cells can be appropriately cultured.

### <Substrate>

By discharging the Liquid A and the Liquid B from a droplet discharging head such as an ink jet head onto a substrate, and then allowing gelation, a three-dimensional structure can be prepared. The size, shape, structure, material, and the like of the substrate is not limited as long as the substrate does not inhibit activity and growth of the cells, and may be appropriately selected depending on the purpose.

The size of the substrate is not limited, and may be appropriately selected depending on the purpose. The shape of the substrate is not limited, and may be appropriately selected depending on the purpose. Examples of the shape include three-dimensional shapes such as dishes, multiplates, flasks, and cell inserts; planar shapes such as glass plates, slide glasses, and cover glasses; and flat membrane shapes.

The structure of the substrate is not limited, and may be appropriately selected depending on the purpose. Examples of the structure include porous structures, mesh structures, irregular structures, and honeycomb structures.

Examples of the material of the substrate include organic materials and inorganic materials. Any one type of these materials may be used individually, or two or more of these materials may be used in combination.

The organic materials are not limited, and may be appropriately selected depending on the purpose. Examples of the organic materials include polyethylene terephthalate (PET), polystyrene (PS), polycarbonate (PC), triacetyl cellulose (TAC), polyimide (PI), nylon (Ny), low-density polyethylene (LDPE), medium-density polyethylene (MDPE), polyvinyl chloride, polyvinylidene chloride, polyphenylene sulfide, polyether sulfone, polyethylene naphthalate, polypropylene, acrylic materials such as urethane acrylate, and cellulose.

The inorganic materials are not limited, and may be appropriately selected depending on the purpose. Examples of the inorganic materials include glasses and ceramics.

### <Ink Jet Method>

Examples of the ink jet method (droplet discharging method) include the on-demand method and the continuous method. The continuous method requires idle discharge before the discharge state becomes stable, and also requires control of the droplet volume. Moreover, in this method, droplet formation continues even during movement among the wells of the well plate. Because of these and other reasons, the dead volume of the suspension used tends to be large. Thus, the on-demand method is more preferred compared to the continuous method.

Examples of the on-demand method include: a plurality of known methods such as the electrostatic method, in which a droplet is formed by drawing of the droplet by electrostatic attraction; the pressure application method, in which a pressure is applied to a liquid to discharge the liquid; and the thermal method, in which a liquid is discharged by film boiling caused by heating. Among these, the pressure application method is preferred from the following reasons.

The electrostatic method requires an electrode placed opposite to a discharge site where a suspension is retained and a droplet is formed. However, for increasing the degree of freedom of the substrate constitution, such placement of the electrode is not preferred.

In the thermal method, heat is locally generated. The heat may affect the polymer components used for the liquid set of the present invention, and may also affect the cells, which are a biomaterial. There is also a concern that burning (kogation) may occur on a heater. Since the heat has different effects depending on the contents and the intended use of the substrate, the thermal method does not necessarily need to be avoided. However, from the viewpoint of the fact that there is no concern of burning on the heater, the pressure application method is more preferred compared to the thermal method.

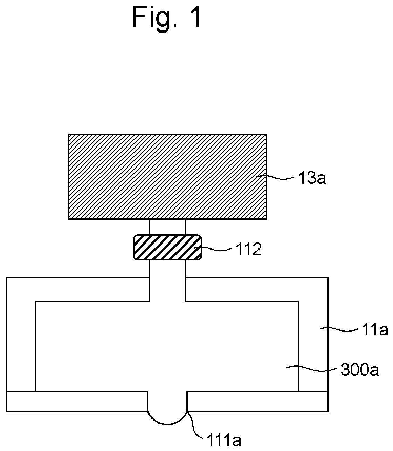

Examples of the pressure application method include a method in which a piezo element is used to apply pressure to a liquid, and a method in which a valve such as an electromagnetic valve is used to apply pressure. FIGs. 1 to 3 illustrate configuration examples of droplet discharging apparatuses that can be used for discharging of droplets.

FIG. 1 is a schematic diagram illustrating an example of an electromagnetic-valve-type discharging head. The electromagnetic-valve-type discharging head includes an electric motor 13a, an electromagnetic valve 112, a liquid chamber wall 11a constituting a liquid chamber as a liquid-storing unit, a liquid 300a, and a nozzle 111a. As the electromagnetic-valve-type discharging head, a dispenser manufactured by TechElan LLC is applicable.

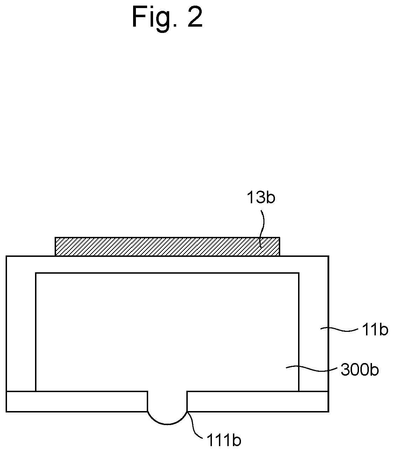

FIG. 2 is a schematic diagram illustrating an example of a piezo-type discharging head. The piezo-type discharging head includes a piezoelectric element 13b, a liquid chamber wall 11b constituting a liquid chamber as a liquid-storing unit, a liquid 300b, and a nozzle 111b. Examples of piezo-type discharging heads include a single-cell printer manufactured by Cytena GmbH.

Although any of these discharging heads can be used, a pressure application method utilizing an electromagnetic valve is incapable of rapidly and repeatedly forming droplets since the droplets are formed by continuous extrusion. Therefore, precise formation of a three-dimensional structure is impossible. Thus, the piezo method is preferably used since the method enables both precise formation of a three-dimensional structure and an improved production throughput.

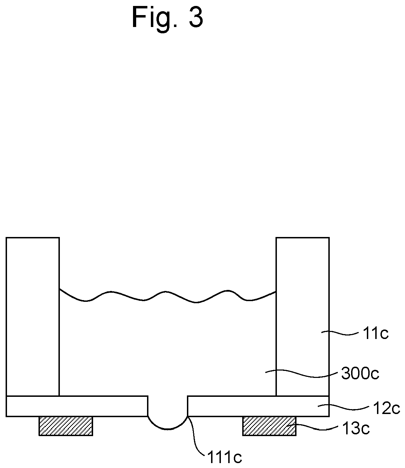

In cases where a liquid containing cells is used, a piezo-type discharging head using a common piezoelectric element 13b may cause the problems of unevenness of the cell density due to sedimentation of the cells, and clogging of the nozzle. In view of this, the constitution illustrated in FIG. 3 is one example of a more preferred constitution. FIG. 3 is a schematic diagram illustrating an example of modification of the piezo-type discharging head using a piezoelectric element in FIG. 2. The discharging head in FIG. 3 includes a piezoelectric element 13c, a liquid chamber wall 11c constituting a liquid chamber as a liquid-storing unit, a liquid 300c, and a nozzle 111c.

In the discharging head of FIG. 3, a voltage from a control unit not illustrated in the figure is applied to the piezoelectric element 13c, to apply compressive stress in the lateral direction of the drawing sheet to deform a membrane 12c in the longitudinal direction of the drawing sheet.

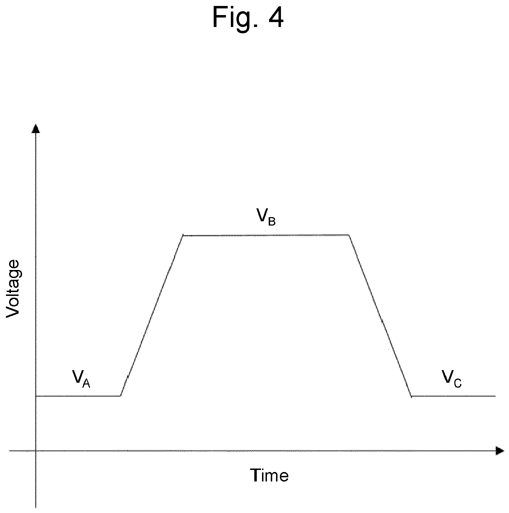

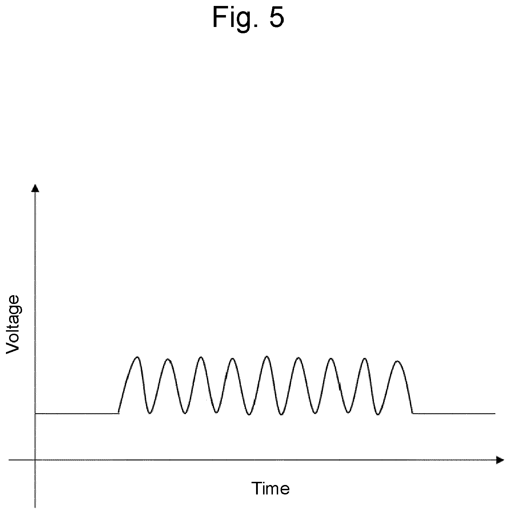

FIG. 4 is a schematic diagram illustrating an example of a voltage applied to a piezoelectric element. FIG. 5 is a schematic diagram illustrating another example of a voltage applied to a piezoelectric element. FIG. 4 illustrates a driving voltage for formation of a droplet. By application of different voltages (V_{A}, V_{B}, and V_{C}), a droplet can be formed. FIG. 5 illustrates a voltage for stirring of the liquid (ink or suspension) without discharging of a droplet. More specifically, during a period without discharge of a droplet, the liquid in the liquid chamber can be stirred by inputting a plurality of pulses that are not strong enough to cause discharge of a droplet. Thus, the density distribution caused by sedimentation of the cells can be suppressed.

By discharging Liquid A and Liquid B onto a substrate using an ink jet head, and then allowing gelation, a three-dimensional structure can be formed. Liquid A and Liquid B may be separately discharged onto the substrate, and may then be mixed together on the substrate to allow gelation. Alternatively, Liquid A and Liquid B may be mixed together immediately before discharging, and may then be discharged before gelation, followed by allowing completion of the gelation reaction to form a three-dimensional structure. In cases where Liquid A and Liquid B are separately discharged, the discharge of Liquid A and Liquid B may be further followed by repeating, a plurality of times, the step of discharging Liquid A and Liquid B.

### EXAMPLES

The present invention is described below more concretely by way of Examples and Comparative Examples. However, the present invention is not limited to these Examples.

### [Example 1]

### • Preparation of Buffer

A buffer was prepared by dissolving 1.15 g of anhydrous disodium hydrogen phosphate (Product No. 197-02865; manufactured by Wako Pure Chemical Industries, Ltd.) and 0.228 g of sodium dihydrogen phosphate (Product No. 197-09705; manufactured by Wako Pure Chemical Industries, Ltd.) in 100 mL of ultrapure water. The pH was 7.4.

### • Preparation of Liquid A

In 2 mL of the buffer, 0.39 g of Tetra-PEG-maleimidyl containing maleimidyl groups at the ends (trade name, SUNBRIGHT PTE-100MA; manufactured by Yuka Sangyo Co., Ltd; weight average molecular weight, 10,000) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-maleimidyl is 19.5% by mass.

### • Preparation of Liquid B

In 2 mL of the buffer, 0.39 g of Tetra-PEG-SH containing thiol groups at the ends (trade name, SUNBRIGHT PTE-100SH; manufactured by Yuka Sangyo Co., Ltd; weight average molecular weight, 10,000) was dissolved, and the resulting solution was filtered through a filter having an average pore size of 0.2 µm (trade name, Minisart Syringe Filter 175497K; manufactured by Sartorius), to prepare Liquid B in which the concentration of Tetra-PEG-SH is 19.5% by mass.

### • Culture of Cells

In an incubator (trade name, KM-CC17RU2; manufactured by Panasonic Corporation; 37°C, environment of 5% by volume CO₂), commercially available normal human skin fibroblasts (trade name, CC2507; manufactured by Lonza; which may be hereinafter also referred to as "NHDF") were cultured for 72 hours in four 100-mm dishes using Dulbecco's Modified Eagle's Medium (trade name, DMEM (IX); manufactured by Life Technologies; which may be hereinafter also referred to as "DMEM") supplemented with 10% by mass fetal bovine serum (which may be hereinafter also referred to as "FBS") and 1% by mass antibiotic (Antibiotic-Antimycotic Mixed Stock Solution (100x); manufactured by Nacalai Tesque, Inc.).

### • Staining of Cells

Green fluorescent dye (trade name, Cell Tracker Green; manufactured by Life Technologies) that had been stored frozen was thawed and allowed to warm to room temperature (25°C). The dye was then dissolved at a concentration of 10 mmol/L (mM) in dimethyl sulfoxide (which may be hereinafter also referred to as "DMSO"). The resulting solution was mixed with FBS-free DMEM to prepare FBS-free DMEM containing the green fluorescent dye at a concentration of 10 µmol/L (µM). Subsequently, 5 mL/dish of the FBS-free DMEM containing the green fluorescent dye was added to the four dishes containing the cultured NHDF, followed by staining in an incubator for 30 minutes. Thereafter, the supernatant was removed using an aspirator. To the dishes, 5 mL/dish of Dulbecco's phosphate buffered saline (trade name, DPBS (IX); manufactured by Life Technologies; which may be hereinafter also referred to as "DPBS") was added, and then the DPBS was removed by aspiration using an aspirator, to wash the surface. After performing two times of the washing operation using DPBS, 2 mL/dish of 0.05% by mass trypsin-0.05% by mass EDTA solution (manufactured by Life Technologies) was added, and the dishes were then incubated in an incubator for 5 minutes to detach cells from the dishes. After confirmation of the detachment of the cells under a phase contrast microscope (apparatus name, CKX41; manufactured by Olympus Corporation), 4 mL of DMEM supplemented with FBS was added to each dish to deactivate the trypsin. The cell suspensions in the four dishes were combined and transferred into one 50-mL centrifuge tube, and centrifugation (trade name, H-19FM; manufactured by KOKUSAN Co., Ltd.; 1.2 × 10³ rpm, 5 minutes, 5°C) was carried out, followed by removal of the supernatant using an aspirator. Thereafter, 2 mL of DMEM supplemented with FBS was added to the centrifuge tube, and gentle pipetting was carried out to disperse the cells, to obtain a cell suspension. From the cell suspension, a 20-µL aliquot was taken into an Eppendorf tube, and 20 µL of 0.4% by mass trypan blue staining solution was added to the tube, followed by pipetting. From the stained cell suspension, a 20-µL aliquot was taken and placed on a PMMA plastic slide. The cell number was counted using a cell counter (trade name, Countess Automated Cell Counter; manufactured by Invitrogen), to determine the cell number in the suspension.

### • Preparation of Stained Cell Suspension

Part of the stained cell suspension was transferred into an Eppendorf tube, and centrifugation (apparatus name, MiniSpin; manufactured by Eppendorf AG; 2.5 × 10³ rpm, 1 minute) was carried out, followed by removal of the supernatant using a pipette. Thereafter, FBS-free DMEM was added to obtain a stained cell suspension having a cell density of 1 × 10⁶ cells/mL.

### • Providing of Substrate

Using Sylgard 184 SILICONE Elastomer Kit 0.5 kg KIT (Product No. 4019862; manufactured by DOW SILICONES CORPORATION), a silicone rubber (PDMS) plate having a thickness of 200 µm was prepared. A piece of 1 cm × 1 cm was excised from the PDMS obtained.

On a cover glass of 18 mm × 18 mm (trade name, No. 1; thickness, from 0.13 to 0.17 mm; manufactured by Matsunami Glass Ind., Ltd.), the PDMS plate of 1 cm × 1 cm was placed such that no air was introduced therebetween. The cover glass and the PDMS plate were placed in a 3.5-cm dish (Product No. 3000-035; manufactured by AGC TECHNO GLASS Co., Ltd.). The 3.5-cm dish was placed in a safety cabinet, and then irradiated with UV light for 15 minutes for sterilization, to provide a substrate.

### • Preparation of Gel

To each of the Liquid A and the Liquid B, 8 µL of a propidium iodide (PI) solution (Product No. P1304MP; manufactured by Thermo Fisher Scientific Inc.) was added. Using the piezo-type discharging head of FIG. 3 (nozzle diameter, 100 µm), 50 drops of the Liquid A containing the PI solution were discharged to the same position on the substrate at a discharging frequency of 100 Hz, and then 50 drops of the Liquid B containing the PI solution were discharged thereto, followed by leaving the liquids to stand for 1 second. The above operation, that is, the discharge of the Liquid A containing the PI solution, the discharge of the Liquid B containing the PI solution, and the leaving of the liquids to stand for 1 second, was repeated 140 times to prepare a dome-shaped gel on the substrate.

### • Arrangement of Cells on Gel Surface

Using the piezo-type cell discharging head of FIG. 3 (nozzle diameter, 100 µm), 6000 drops of the stained cell suspension were discharged to the surface of the dome-shaped gel to arrange 3000 cells on the surface of the gel. Thereafter, 3 mL of DMEM supplemented with FBS was quickly added to the 3.5-cm dish using a micropipette, and the dish was then placed in an incubator (37°C, environment of 5% by volume CO₂). Thus, a "gel having cells arranged on the surface" was prepared.

### • Preparation of Unstained Cell Suspension

Cells were cultured by the same method as described above to prepare a cell suspension without cell staining.

### • Preparation of Liquid A Containing Suspended Cells

Into an Eppendorf tube, 125 µL of the unstained cell suspension was transferred, and centrifugation (2.5 × 10³ rpm, 1 minute) was carried out, followed by removal of the supernatant using a micropipette. Into the Eppendorf tube, 250 µL of Liquid A was added to prepare Liquid A containing Tetra-PEG-maleimidyl at a concentration of 19.5% by mass, and also containing cells suspended therein at a density of 5 × 10⁵ cells/mL.

### • Preparation of Liquid B Containing Suspended Cells

Similarly to Liquid A, 250 µL of Liquid B was added into the Eppendorf tube to prepare Liquid B containing Tetra-PEG-SH at a concentration of 19.5% by mass, and also containing cells suspended therein at a density of 5 × 10⁵ cells/mL.

### • Preparation of Gel Containing Three-Dimensionally Layered Cells Therein Using Ink Jet Head

By the same method as described above, a substrate was provided. A gel was prepared in the same manner except that the Liquid A containing suspended cells was used instead of the Liquid A in "Arrangement of Cells on Gel Surface", and that the Liquid B containing suspended cells was used instead of the Liquid B in "Arrangement of Cells on Gel Surface". Thereafter, 3 mL of DMEM was quickly added to the 3.5-cm dish using a micropipette, and the dish was then placed in an incubator (37°C, environment of 5% by volume CO₂), followed by carrying out culture for 24 hours. Thus, a "gel containing three-dimensionally layered cells therein" was prepared using the inkjet head.

### [Example 2]

### • Preparation of Buffer

A buffer was prepared by dissolving 0.76 g of anhydrous disodium hydrogen phosphate and 0.43 g of anhydrous citric acid (Product No. 030-05525; manufactured by Wako Pure Chemical Industries, Ltd.) in 100 mL of ultrapure water. The pH was 5.2.

### • Preparation of Liquid A

In 2 mL of the buffer, 0.007 g of Tetra-PEG-maleimidyl was dissolved, and the resulting solution was filtered through a filter having an average pore size of 0.2 µm, to prepare Liquid A in which the concentration of Tetra-PEG-maleimidyl is 0.35% by mass.

### • Preparation of Liquid B

In 2 mL of the buffer, 0.007 g of Tetra-PEG-SH was dissolved, and the resulting solution was filtered through a filter having an average pore size of 0.2 µm, to prepare Liquid B in which the concentration of Tetra-PEG-SH is 0.35% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 3]

### • Preparation of Buffer

A buffer was prepared by dissolving 0.94 g of anhydrous disodium hydrogen phosphate and 0.32 g of anhydrous citric acid in 100 mL of ultrapure water. The pH was 6.2.

### • Preparation of Liquid A

In 2 mL of the buffer, 0.036 g of Tetra-PEG-maleimidyl was dissolved, and the resulting solution was filtered through a filter having an average pore size of 0.2 µm, to prepare Liquid A in which the concentration of Tetra-PEG-maleimidyl is 1.8% by mass.

### • Preparation of Liquid B

In 2 mL of the buffer, 0.036 g of Tetra-PEG-SH was dissolved, and the resulting solution was filtered through a filter having an average pore size of 0.2 µm, to prepare Liquid B in which the concentration of Tetra-PEG-SH is 1.8% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 4]

### • Preparation of Buffer

A buffer was prepared by dissolving 0.86 g of anhydrous disodium hydrogen phosphate and 0.38 g of anhydrous citric acid in 100 mL of ultrapure water. The pH was 5.8.

### • Preparation of Liquid A

In 2 mL of the buffer, 0.032 g of Tetra-PEG-maleimidyl was dissolved, and the resulting solution was filtered through a filter having an average pore size of 0.2 µm, to prepare Liquid A in which the concentration of Tetra-PEG-maleimidyl is 1.6% by mass.

### • Preparation of Liquid B

In 2 mL of the buffer, 0.032 g of Tetra-PEG-SH was dissolved, and the resulting solution was filtered through a filter having an average pore size of 0.2 µm, to prepare Liquid B in which the concentration of Tetra-PEG-SH is 1.6% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 5]

### • Preparation of Buffer

A buffer was prepared by dissolving 0.11 g of sodium hydroxide (Product No. 1310-73-2; manufactured by Wako Pure Chemical Industries, Ltd.) and 0.375 g of glycine (Product No. 073-00732; manufactured by Wako Pure Chemical Industries, Ltd.) in 100 mL of ultrapure water. The pH was 9.8.

### • Preparation of Liquid A and Liquid B

Liquid A and Liquid B were prepared in the same manner as in Example 1 except that the buffer at pH 9.8 was used instead of the buffer in Example 1.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 6]

### • Preparation of Buffer

A buffer was prepared in the same manner as in Example 2. The pH was 5.2.

### • Preparation of Liquid A and Liquid B

Liquid A and Liquid B were prepared in the same manner as in Example 1 except that the buffer at pH 5.2 was used instead of the buffer in Example 1.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 7]

### • Preparation of Buffer

A buffer was prepared in the same manner as in Example 5. The pH was 9.8.

### • Preparation of Liquid A and Liquid B

Liquid A and Liquid B were prepared in the same manner as in Example 2 except that the buffer at pH 9.8 was used instead of the buffer in Example 2.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 8]

### • Preparation of Buffer

A buffer was prepared in the same manner as in Example 5. The pH was 9.8.

### • Preparation of Liquid A and Liquid B

Liquid A and Liquid B were prepared in the same manner as in Example 3 except that the buffer at pH 9.8 was used instead of the buffer in Example 3.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 9]

### • Preparation of Buffer

A buffer was prepared in the same manner as in Example 1. The pH was 7.4.

### • Preparation of Liquid A and Liquid B

The same operation as in Example 1 was carried out except that the weight of Tetra-PEG-maleimidyl was 0.116 g, and that the weight of Tetra-PEG-SH was 0.116 g. Thus, Liquid A containing Tetra-PEG-maleimidyl at a concentration of 5.8% by mass, and Liquid B containing Tetra-PEG-SH at a concentration of 5.8% by mass, were prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 10]

### • Preparation of Buffer

A buffer was prepared in the same manner as in Example 1. The pH was 7.4.

### • Preparation of Liquid A and Liquid B

The same operation as in Example 1 was carried out except that the weight of Tetra-PEG-maleimidyl was 0.124 g, and that the weight of Tetra-PEG-SH was 0.124 g. Thus, Liquid A containing Tetra-PEG-maleimidyl at a concentration of 6.2% by mass, and Liquid B containing Tetra-PEG-SH at a concentration of 6.2% by mass, were prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 11]

### • Preparation of Buffer

A buffer was prepared in the same manner as in Example 2. The pH was 5.2.

### • Preparation of Thrombin Liquid

Thrombin (trade name, Thrombin from bovine Plasma; manufactured by Sigma-Aldrich) was diluted with DPBS to 200 U/mL, to prepare a thrombin liquid.

### • Preparation of Liquid A and Liquid B

After preparing Liquid A and Liquid B in the same manner as in Example 2, 0.02 g of fibrinogen (trade name, Fibrinogen from bovine plasma; manufactured by Sigma-Aldrich) was added to Liquid A, and the fibrinogen was dissolved using a microtube rotator (Product No. MTR-103; manufactured by Ai'ris Corporation). To Liquid B, 50 µL of the thrombin liquid was added. Thus, Liquid A containing Tetra-PEG-maleimidyl at a concentration of 0.35% by mass, and also containing fibrinogen at a concentration of 1.0% by mass, was prepared. In addition, Liquid B containing Tetra-PEG-SH at a concentration of 0.35% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 12]

### • Preparation of Buffer

A buffer was prepared in the same manner as in Example 2. The pH was 5.2.

### • Preparation of Liquid A and Liquid B

After preparing Liquid A and Liquid B in the same manner as in Example 2, 20 µL of Matrigel stock solution (Product No. 354234, manufactured by Corning) was added to each liquid. Thus, Liquid A containing Tetra-PEG-maleimidyl at a concentration of 0.35% by mass, and also containing Matrigel at a concentration of 0.1% by mass, was prepared. In addition, Liquid B containing Tetra-PEG-SH at a concentration of 0.35% by mass, and also containing Matrigel at a concentration of 0.1% by mass, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 13]

### • Preparation of Buffer

A buffer was prepared in the same manner as in Example 1. The pH was 7.4.

### • Preparation of Liquid A and Liquid B

After preparing Liquid A and Liquid B in the same manner as in Example 9, 0.02 g of fibrinogen was added to Liquid A, and the fibrinogen was dissolved using a microtube rotator. To Liquid B, 50 µL of the thrombin liquid described in Example 11 was added. Thus, Liquid A containing Tetra-PEG-maleimidyl at a concentration of 5.8% by mass, and also containing fibrinogen at a concentration of 1.0% by mass, was prepared. In addition, Liquid B containing Tetra-PEG-SH at a concentration of 5.8% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 14]

### • Preparation of Buffer

A buffer was prepared in the same manner as in Example 1. The pH was 7.4.

### • Preparation of Liquid A and Liquid B

After preparing Liquid A and Liquid B in the same manner as in Example 9, 20 µL of Matrigel stock solution was added to each liquid. Thus, Liquid A containing Tetra-PEG-maleimidyl at a concentration of 5.8% by mass, and also containing Matrigel at a concentration of 0.1% by mass, was prepared. In addition, Liquid B containing Tetra-PEG-SH at a concentration of 5.8% by mass, and also containing Matrigel at a concentration of 0.1% by mass, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 15]

### • Preparation of Buffer

A buffer was prepared in the same manner as in Example 1. The pH was 7.4.

### • Preparation of Liquid A and Liquid B

After preparing Liquid A and Liquid B in the same manner as in Example 10, 0.02 g of fibrinogen was added to Liquid A, and the fibrinogen was dissolved using a microtube rotator. To Liquid B, 50 µL of the thrombin liquid described in Example 11 was added. Thus, Liquid A containing Tetra-PEG-maleimidyl at a concentration of 6.2% by mass, and also containing fibrinogen at a concentration of 1.0% by mass, was prepared. In addition, Liquid B containing Tetra-PEG-SH at a concentration of 6.2% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 16]

### • Preparation of Buffer

A buffer was prepared by dissolving 0.73 g of anhydrous disodium hydrogen phosphate and 0.47 g of anhydrous citric acid in 100 mL of ultrapure water. The pH was 5.0.

### • Preparation of Liquid A

In 2 mL of the buffer, 0.006 g of Tetra-PEG-maleimidyl was dissolved, and the resulting solution was filtered through a filter having an average pore size of 0.2 µm, to prepare Liquid A in which the concentration of Tetra-PEG-maleimidyl is 0.3% by mass.

### • Preparation of Liquid B

In 2 mL of the buffer, 0.006 g of Tetra-PEG-SH was dissolved, and the resulting solution was filtered through a filter having an average pore size of 0.2 µm, to prepare Liquid B in which the concentration of Tetra-PEG-SH is 0.3% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 17]

### • Preparation of Buffer

A buffer was prepared in the same manner as in Example 16. The pH was 5.0.

### • Preparation of Liquid A and Liquid B

The same operation as in Example 16 was carried out except that the weight of Tetra-PEG-maleimidyl was 0.08 g, and that the weight of Tetra-PEG-SH was 0.08 g. Thus, Liquid A containing Tetra-PEG-maleimidyl at a concentration of 4.0% by mass, and Liquid B containing Tetra-PEG-SH at a concentration of 4.0% by mass, were prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 18]

### • Preparation of Buffer

A buffer was prepared by dissolving 0.13 g of sodium hydroxide and 0.375 g of glycine in 100 mL of ultrapure water. The pH was 10.0.

### • Preparation of Liquid A

In 2 mL of the buffer, 0.08 g of Tetra-PEG-maleimidyl was dissolved, and the resulting solution was filtered through a filter having an average pore size of 0.2 µm, to prepare Liquid A in which the concentration of Tetra-PEG-maleimidyl is 4.0% by mass.

### • Preparation of Liquid B

In 2 mL of the buffer, 0.08 g of Tetra-PEG-SH was dissolved, and the resulting solution was filtered through a filter having an average pore size of 0.2 µm, to prepare Liquid B in which the concentration of Tetra-PEG-SH is 4.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 19]

### • Preparation of Buffer

A buffer was prepared in the same manner as in Example 18. The pH was 10.0.

### • Preparation of Liquid A and Liquid B

The same operation as in Example 18 was carried out except that the weight of Tetra-PEG-maleimidyl was 0.006 g, and that the weight of Tetra-PEG-SH was 0.006 g. Thus, Liquid A containing Tetra-PEG-maleimidyl at a concentration of 0.3% by mass, and Liquid B containing Tetra-PEG-SH at a concentration of 0.3% by mass, were prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 20]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.40 g, and that the concentration of each liquid was therefore 20.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 21]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.08 g, and that the concentration of each liquid was therefore 4.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 22]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.04 g, and that the concentration of each liquid was therefore 2.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 23]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.02 g, and that the concentration of each liquid was therefore 1.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 24]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.006 g, and that the concentration of each liquid was therefore 0.3% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 25]

### • Preparation of Buffer

A buffer was prepared by dissolving 2.69 g of anhydrous disodium hydrogen phosphate and 0.13 g of anhydrous sodium dihydrogen phosphate in 100 mL of ultrapure water. The pH was 8.0.

### • Preparation of Liquid A

In 2 mL of the buffer, 0.08 g of Tetra-PEG-maleimidyl was dissolved, and the resulting solution was filtered through a filter having an average pore size of 0.2 µm, to prepare Liquid A in which the concentration of Tetra-PEG-maleimidyl is 4.0% by mass.

### • Preparation of Liquid B

In 2 mL of the buffer, 0.08 g of Tetra-PEG-SH was dissolved, and the resulting solution was filtered through a filter having an average pore size of 0.2 µm, to prepare Liquid B in which the concentration of Tetra-PEG-SH is 4.0% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 26]

### • Preparation of Buffer

A buffer was prepared in the same manner as in Example 25. The pH was 8.0.

### • Preparation of Liquid A and Liquid B

Liquid A and Liquid B were prepared in the same manner as in Example 25 except that the weight of each of Tetra-PEG-maleimidyl and Tetra-PEG-SH was 0.006 g, and that the concentration of each liquid was therefore 0.3% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 27]

### • Preparation of Liquid A and Liquid B

Liquid A and Liquid B were prepared in the same manner as in Example 22 except that DMEM was used instead of the buffer in Example 22. The pH was 7.4.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 28]

### • Preparation of Liquid A and Liquid B

Liquid A and Liquid B were prepared in the same manner as in Example 23 except that DMEM was used instead of the buffer in Example 23. The pH was 7.4.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 29]

### • Preparation of Buffer

A buffer was prepared in the same manner as in Example 1. The pH was 7.4.

### • Preparation of Liquid A and Liquid B

After preparing Liquid A and Liquid B in the same manner as in Example 22, 0.002 g of fibrinogen was added to Liquid A, and the fibrinogen was dissolved using a microtube rotator. To Liquid B, 50 µL of the thrombin liquid described in Example 11 was added. Thus, Liquid A containing Tetra-PEG-maleimidyl at a concentration of 2.0% by mass, and also containing fibrinogen at a concentration of 0.1% by mass, was prepared. In addition, Liquid B containing Tetra-PEG-SH at a concentration of 2.0% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 30]

### • Preparation of Liquid A and Liquid B

Liquid A and Liquid B were prepared in the same manner as in Example 29 except that the amount of the fibrinogen added was 0.02 g. Thus, Liquid A containing Tetra-PEG-maleimidyl at a concentration of 2.0% by mass, and also containing fibrinogen at a concentration of 1.0% by mass, was prepared. In addition, Liquid B containing Tetra-PEG-SH at a concentration of 2.0% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 31]

### • Preparation of Liquid A and Liquid B

Liquid A and Liquid B were prepared in the same manner as in Example 29 except that the amount of the fibrinogen added was 0.03 g. Thus, Liquid A containing Tetra-PEG-maleimidyl at a concentration of 2.0% by mass, and also containing fibrinogen at a concentration of 1.5% by mass, was prepared. In addition, Liquid B containing Tetra-PEG-SH at a concentration of 2.0% by mass, and also containing thrombin at a concentration of 5 U/mL, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 32]

### • Preparation of Buffer

A buffer was prepared in the same manner as in Example 1. The pH was 7.4.

### • Preparation of Liquid A and Liquid B

After preparing Liquid A and Liquid B in the same manner as in Example 22, 10 µL of Matrigel stock solution was added to each liquid. Thus, Liquid A containing Tetra-PEG-maleimidyl at a concentration of 2.0% by mass, and also containing Matrigel at a concentration of 0.05% by mass, was prepared. In addition, Liquid B containing Tetra-PEG-SH at a concentration of 2.0% by mass, and also containing Matrigel at a concentration of 0.05% by mass, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 33]

### • Preparation of Liquid A and Liquid B

Liquid A and Liquid B were prepared in the same manner as in Example 32 except that the amount of the Matrigel stock solution added was 20 µL. Thus, Liquid A containing Tetra-PEG-maleimidyl at a concentration of 2.0% by mass, and also containing Matrigel at a concentration of 0.1% by mass, was prepared. In addition, Liquid B containing Tetra-PEG-SH at a concentration of 2.0% by mass, and also containing Matrigel at a concentration of 0.1% by mass, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Example 34]

### • Preparation of Liquid A and Liquid B

Liquid A and Liquid B were prepared in the same manner as in Example 32 except that the amount of the Matrigel stock solution added was 200 µL. Thus, Liquid A containing Tetra-PEG-maleimidyl at a concentration of 2.0% by mass, and also containing Matrigel at a concentration of 1.0% by mass, was prepared. In addition, Liquid B containing Tetra-PEG-SH at a concentration of 2.0% by mass, and also containing Matrigel at a concentration of 1.0% by mass, was prepared.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were carried out in the same manner as in Example 1.

### [Comparative Example 1]

### • Preparation of Buffer

A buffer was prepared in the same manner as in Example 1. The pH was 7.4.

### • Preparation of Liquid A and Liquid B

Liquid A and Liquid B were prepared in the same manner as in Example 1 except that the weight of each of Tetra-PEG-maleimidyl and Tetra-PEG-SH was 0.41 g, and that the concentration of each liquid was therefore 20.5% by mass.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were not carried out since Liquid A and Liquid B could not be discharged by the ink jet head.

### [Comparative Example 2]

### • Preparation of Aqueous Sodium Alginate Solution

After dissolving 1.5 g of sodium alginate (trade name, Kimica Algin SKAT-ONE; manufactured by KIMICA Corporation) in 100 mL of ultrapure water, the resulting solution was filtered through a filter having an average pore size of 0.2 µm (trade name, Minisart Syringe Filter 175497K; manufactured by Sartorius), to prepare 1.5% by mass aqueous sodium alginate solution. The aqueous sodium alginate solution was used as Liquid A.

### • Preparation of Aqueous Calcium Chloride Solution

After dissolving 0.584 g of calcium chloride (Product No. 192-13925; manufactured by Wako Pure Chemical Industries, Ltd.) in 100 mL of ultrapure water, the resulting solution was filtered through a filter having an average pore size of 0.2 µm (trade name, Minisart Syringe Filter 175497K; manufactured by Sartorius), to prepare 0.58% by mass aqueous calcium chloride solution. The aqueous calcium chloride solution was used as Liquid B.

Cell culture, cell staining, preparation of a stained cell suspension, providing of a substrate, preparation of a gel, and arrangement of cells on the gel surface were carried out in the same manner as in Example 1. Although the shape of the "gel having cells arranged on the surface" could not be maintained for 24 hours, preparation of an unstained cell suspension, preparation of Liquid A containing suspended cells, preparation of Liquid B containing suspended cells, and preparation of a gel containing three-dimensionally layered cells therein were also carried out in the same manner as in Example 1. Detachment of the "gel containing three-dimensionally layered cells therein" from the substrate occurred during the 24-hour culture, and the size of the gel was found to have been reduced to about half relative to the size found immediately after its preparation.

### [Comparative Example 3]

### • Preparation of Aqueous Sodium Alginate Solution and Aqueous Calcium Chloride Solution

An aqueous sodium alginate solution (Liquid A) and an aqueous calcium chloride solution (Liquid B) were prepared in the same manner as in Comparative Example 2 except that the weight of sodium alginate was 2.0 g, and that the concentration of the aqueous sodium alginate solution was 2.0% by mass.

Cell staining, preparation of a stained cell suspension, providing of a substrate, preparation of a gel, and arrangement of cells on the gel surface were not carried out since Liquid A and Liquid B could not be discharged by the ink jet head.

For reference, in order to evaluate whether cells can survive in a gel prepared with Liquid A and Liquid B, preparation of an unstained cell suspension, preparation of Liquid A containing suspended cells, and preparation of Liquid B containing suspended cells were carried out in the same manner as in Example 1, and the "gel containing three-dimensionally layered cells therein" was manually prepared.

### • Manual Preparation of Gel Containing Three-Dimensionally Layered Cells Therein

By the same method as in Example 1, a substrate was provided. Using a micropipette, 3.5 µL of Liquid A was added dropwise onto the substrate. Further, using a micropipette, 3.5 µL of Liquid B was added dropwise onto the droplet, and then pipetting was carried out several times to prepare a gel. Thereafter, 3 mL of DMEM was quickly added to the 3.5-cm dish using a micropipette, and the dish was then placed in an incubator (37°C, environment of 5% by volume CO₂), followed by carrying out culture for 24 hours. Thus, a "gel containing three-dimensionally layered cells therein" was manually prepared.

### [Comparative Example 4]

### • Preparation of Fibrinogen Liquid

To 1 mL of DPBS, 0.01 g of fibrinogen was added. The fibrinogen was dissolved using a microtube rotator, to prepare 1.0% by mass fibrinogen liquid. The fibrinogen liquid was used as Liquid A.

### • Preparation of Thrombin Liquid

To 900 µL of DPBS, 100 µL of the 200 U/mL thrombin liquid described in Example 11 was added, to prepare 20 U/mL thrombin liquid. The thrombin liquid was used as Liquid B.

Cell culture, cell staining, preparation of a stained cell suspension, providing of a substrate, preparation of a gel, and arrangement of cells on the gel surface were carried out in the same manner as in Example 1. Since all cells precipitated in the "gel having cells arranged on the surface", three-dimensional arrangement of the cells was impossible. Therefore, preparation of an unstained cell suspension, preparation of Liquid A containing suspended cells, preparation of Liquid B containing suspended cells, and preparation of a gel containing three-dimensionally layered cells therein were not carried out.

### [Comparative Example 5]

### • Preparation of Fibrinogen Liquid and Thrombin Liquid

A fibrinogen liquid (Liquid A) and a thrombin liquid (Liquid B) were prepared in the same manner as in Comparative Example 4 except that the weight of fibrinogen was 0.02 g.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were not carried out since Liquid A and Liquid B could not be discharged by the ink jet head.

### [Comparative Example 6]

### • Preparation of Buffer

A buffer was prepared by dissolving 0.70 g of anhydrous disodium hydrogen phosphate and 0.49 g of anhydrous citric acid in 100 mL of ultrapure water. The pH was 4.8.

### • Preparation of Liquid A

In 2 mL of the buffer, 0.005 g of Tetra-PEG-maleimidyl was dissolved, and the resulting solution was filtered through a filter having an average pore size of 0.2 µm, to prepare Liquid A in which the concentration of Tetra-PEG-maleimidyl is 0.25% by mass.

### • Preparation of Liquid B

In 2 mL of the buffer, 0.005 g of Tetra-PEG-SH was dissolved, and the resulting solution was filtered through a filter having an average pore size of 0.2 µm, to prepare Liquid B in which the concentration of Tetra-PEG-SH is 0.25% by mass.

Cell culture, cell staining, preparation of a stained cell suspension, providing of a substrate, preparation of a gel, and arrangement of cells on the gel surface were carried out in the same manner as in Example 1. Since not less than half of the cells precipitated in the "gel having cells arranged on the surface", three-dimensional arrangement of the cells was impossible. Therefore, preparation of an unstained cell suspension, preparation of Liquid A containing suspended cells, preparation of Liquid B containing suspended cells, and preparation of a gel containing three-dimensionally layered cells therein were not carried out.

### [Comparative Example 7]

### • Preparation of Buffer

A buffer was prepared by dissolving 0.50 g of anhydrous disodium hydrogen phosphate and 0.62 g of anhydrous citric acid in 100 mL of ultrapure water. The pH was 3.8.

### • Preparation of Liquid A

In 2 mL of the buffer, 0.014 g of Tetra-PEG-maleimidyl was dissolved, and the resulting solution was filtered through a filter having an average pore size of 0.2 µm, to prepare Liquid A in which the concentration of Tetra-PEG-maleimidyl is 0.7% by mass.

### • Preparation of Liquid B

In 2 mL of the buffer, 0.014 g of Tetra-PEG-SH was dissolved, and the resulting solution was filtered through a filter having an average pore size of 0.2 µm, to prepare Liquid B in which the concentration of Tetra-PEG-SH is 0.7% by mass.

Cell culture, cell staining, preparation of a stained cell suspension, providing of a substrate, preparation of a gel, and arrangement of cells on the gel surface were carried out in the same manner as in Example 1. Since not less than half of the cells precipitated in the "gel having cells arranged on the surface", three-dimensional arrangement of the cells was impossible. Therefore, preparation of an unstained cell suspension, preparation of Liquid A containing suspended cells, preparation of Liquid B containing suspended cells, and preparation of a gel containing three-dimensionally layered cells therein were not carried out.

### [Comparative Example 8]

### • Preparation of Buffer

A buffer was prepared by dissolving 0.15 g of sodium hydroxide and 0.375 g of glycine in 100 mL of ultrapure water. The pH was 10.4.

### • Preparation of Liquid A

In 2 mL of the buffer, 0.39 g of Tetra-PEG-maleimidyl was dissolved, and the resulting solution was filtered through a filter having an average pore size of 0.2 µm, to prepare Liquid A in which the concentration of Tetra-PEG-maleimidyl is 19.5% by mass.

### • Preparation of Liquid B

In 2 mL of the buffer, 0.39 g of Tetra-PEG-SH was dissolved, and the resulting solution was filtered through a filter having an average pore size of 0.2 µm, to prepare Liquid B in which the concentration of Tetra-PEG-SH is 19.5% by mass.

Cell culture, cell staining, preparation of a stained cell suspension, providing of a substrate, preparation of a gel, and arrangement of cells on the gel surface were carried out in the same manner as in Example 1. Since the shape of the "gel having cells arranged on the surface" could not be maintained for 24 hours, preparation of an unstained cell suspension, preparation of Liquid A containing suspended cells, preparation of Liquid B containing suspended cells, and preparation of a gel containing three-dimensionally layered cells therein were not carried out.

### [Comparative Example 9]

### • Preparation of Buffer

A buffer was prepared in the same manner as in Example 4. The pH was 5.8.

### • Preparation of Liquid A

In 2 mL of the buffer, 0.005 g of Tetra-PEG-maleimidyl was dissolved, and the resulting solution was filtered through a filter having an average pore size of 0.2 µm, to prepare Liquid A in which the concentration of Tetra-PEG-maleimidyl is 0.25% by mass.

### • Preparation of Liquid B

In 2 mL of the buffer, 0.005 g of Tetra-PEG-SH was dissolved, and the resulting solution was filtered through a filter having an average pore size of 0.2 µm, to prepare Liquid B in which the concentration of Tetra-PEG-SH is 0.25% by mass.

Cell culture, cell staining, preparation of a stained cell suspension, providing of a substrate, preparation of a gel, and arrangement of cells on the gel surface were carried out in the same manner as in Example 1. Since not less than half of the cells precipitated in the "gel having cells arranged on the surface", three-dimensional arrangement of the cells was impossible. Therefore, preparation of an unstained cell suspension, preparation of Liquid A containing suspended cells, preparation of Liquid B containing suspended cells, and preparation of a gel containing three-dimensionally layered cells therein were not carried out.

### [Comparative Example 10]

### • Preparation of Matrigel Liquid

In 20 mL of the buffer described in Example 1, 0.2 g of Matrigel stock solution was dissolved, to prepare 1.0% by mass Matrigel liquid. The Matrigel liquid was used as Liquid A and Liquid B.

Cell culture, cell staining, preparation of a stained cell suspension, providing of a substrate, preparation of a gel, and arrangement of cells on the gel surface were carried out in the same manner as in Example 1. Since not less than half of the cells precipitated in the "gel having cells arranged on the surface", three-dimensional arrangement of the cells was impossible. Therefore, preparation of an unstained cell suspension, preparation of Liquid A containing suspended cells, preparation of Liquid B containing suspended cells, and preparation of a gel containing three-dimensionally layered cells therein were not carried out.

### [Comparative Example 11]

### • Preparation of Matrigel Liquid

In 20 mL of the buffer described in Example 1, 0.4 g of Matrigel stock solution was dissolved, to prepare 2.0% by mass Matrigel liquid. The Matrigel liquid was used as Liquid A and Liquid B.

Cell culture, cell staining, preparation of a stained cell suspension, providing of a substrate, preparation of a gel, arrangement of cells on the gel surface, preparation of an unstained cell suspension, preparation of Liquid A containing suspended cells, preparation of Liquid B containing suspended cells, and preparation of a gel containing three-dimensionally layered cells therein were not carried out since Liquid A and Liquid B could not be discharged by the ink jet head.

### <Measurement of Viscosities of Ink Liquids>

In order to investigate the upper limit of the viscosity of a liquid that can be discharged by an ink jet head, the viscosities of Liquid A and Liquid B in Examples 1, 11 to 15, 20, and 29 to 34, and Comparative Examples 1 to 5, 10, and 11 were measured. Regarding Liquid A and Liquid B in Examples 2 to 10, 16 to 19, and 21 to 28, and Comparative Examples 6 to 9, the concentration of Tetra-PEG-maleimidyl or Tetra-PEG-SH is not more than 20% by mass. Thus, since it is clear that the viscosities in these Examples and Comparative Examples are not more than the viscosities in Example 1, they were not subjected to the measurement. For the Examples and Comparative Examples not subjected to the measurement, the corresponding cells in Table 1 are marked with "-".

For the viscosity measurement, dynamic rotational measurement was carried out under the following conditions using a rheometer manufactured by Anton Paar GmbH. The viscosity (mPa·s) at a shear rate of 1000/s was employed as the viscosity of the liquid. The results are presented in Table 1.
- Apparatus: Physica MCR301
- Cone-plate: CP50-1
- Temperature: 25°C
- Liquid volume: 1 mL
- Variable: shear rate
- Condition of variable: logarithmic increase/decrease
- Range of variable: from 1 to 1000/s
- Measurement points: 13 points
- Measurement interval: fixed to 10 seconds

### <Evaluation of Liquid Discharge by Ink Jet Head>

In order to investigate the upper limit of the viscosity of a liquid that can be discharged by an ink jet head, the discharge performances of Liquid A and Liquid B in Examples 1 to 34 and Comparative Examples 1 to 11 were evaluated using an industrial ink jet head (trade name, MH2420; manufactured by Ricoh Industry Company, Ltd.) and the piezo-type discharging head of FIG. 3 (nozzle diameter, 100 µm). For each liquid, ink droplets that were dropped from the head nozzle were observed.

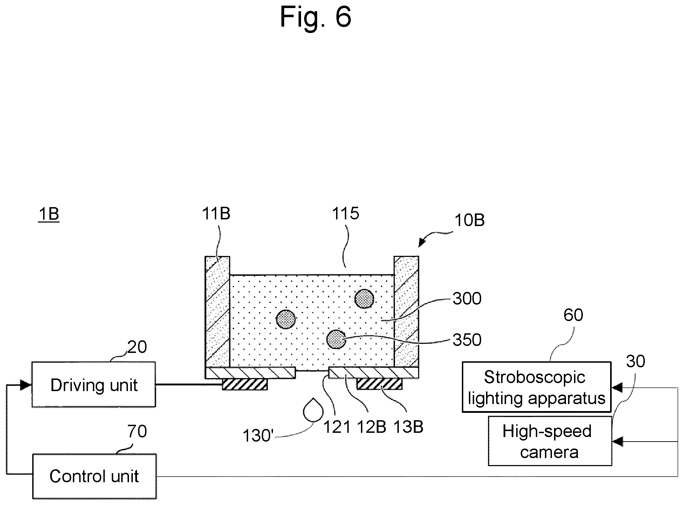

FIG. 6 is a schematic diagram illustrating an ink droplet-observing mechanism 1B. The ink droplet-observing mechanism 1B is provided with a high-speed camera 30 configured to capture an image, from a lateral direction, of a droplet 130' dropped from a head nozzle 121; a stroboscopic lighting apparatus 60 configured to radiate light to the droplet in synchronization with the dropping of the droplet; a control unit 70 configured to control voltage application to a membrane 12B; and a driving unit 20. The timing when the shutter of the high-speed camera 30 opens was synchronized with the timing of the voltage application to the membrane 12B.

The liquid was rated as "good" in cases where discharge of a droplet occurred from each nozzle with an applied voltage within the specified range. The liquid was rated as "poor" in cases where no discharge occurred even with the maximum applied voltage within the specified range, and in cases where no discharge occurred due to clogging or the like in not less than half of the nozzles. The results are presented in Table 1.

From the results in Table 1, it was found that both ink jet heads are capable of discharging a solution containing either Tetra-PEG-maleimidyl or Tetra-PEG-SH in cases where the liquid has a concentration of up to 20% by mass, that is, a viscosity of up to 30 mPa·s. It was also found that both ink jet heads are capable of discharging a sodium alginate liquid whose concentration is up to 1.5% by mass, and a fibrinogen liquid whose concentration is up to 1.0% by mass. Since a fibrinogen solution is a Bingham fluid, it could not be discharged by the MH2420 head even in the case where the liquid had a viscosity of as low as 2.0% by mass. Comparative Examples 1, 3, 5, and 11, in which the discharge was impossible, were not subjected to the following liquid toxicity evaluation test and the later tests.

### <Liquid Toxicity Evaluation>

For each of Examples 1 to 34 and Comparative Examples 2, 4, and 6 to 10, in which the discharge was possible in the evaluation of liquid discharge by the ink jet head, cells were immersed in each ink liquid for 2 hours, and then the cell survival rate was calculated by a WST-1 assay, to evaluate the toxicity of the liquid to the cells.

Each of Liquid A and Liquid B containing suspended cells was left to stand for 2 hours in a 15-mL centrifuge tube. After centrifugation (1.2 × 10³ rpm, 5 minutes, 5°C), the supernatant was removed using an aspirator. Thereafter, 2 mL of DMEM supplemented with FBS was added to the centrifuge tube, and gentle pipetting was carried out to disperse the cells, to obtain a cell suspension again. Into each of 8 wells of a 96-well plate, 200 µL of the cell suspension after the redispersion was placed, and culture was performed in an incubator for 24 hours.

Thereafter, 20 µL of WST-1 (trade name, Premix WST-1 Cell Proliferation Assay System; manufactured by Takara Bio Inc.) was added to each well, and coloring was allowed for 1 hour. Thereafter, each well was subjected to measurement of the absorbances at 450 nm and 620 nm (for reference) using a plate reader (trade name, Cytation 5 Imaging Plate Reader; manufactured by BIOTEC Co., Ltd.), and the ratio between the absorbances at 450 nm and 620 nm was calculated.

The average for the 8 wells was employed as the value for calculation of the survival rate. To obtain a reference value (control) for the survival rate of 100%, a liquid obtained by leaving the cells to stand in DMEM supplemented with FBS at room temperature for 2 hours, and then performing culture for 24 hours was employed. In cases where the survival rate was not less than 75%, the liquid was judged to be non-toxic, and rated as "good". The results are presented in Table 1 (see the "Liquid toxicity" row in Table 1).

From the results in Table 1, it could be confirmed that the liquids are non-toxic to the cells except for the liquids in which the pH of the buffer, that is, the pH of Liquid A or Liquid B, is 3.8 (Comparative Example 7). It could thus be confirmed that the liquid is non-toxic to the cells at least in cases where the concentration of Tetra-PEG-maleimidyl or Tetra-PEG-SH is from 0.3% by mass to 20% by mass and, at the same time, the pH of the buffer, that is, the pH of Liquid A or Liquid B, is from 5 to 10.

### <Evaluation of Three-Dimensional Arrangement of Cells>

For each of Examples 1 to 34 and Comparative Examples 2, 4, and 6 to 10, in which the discharge was possible in the evaluation of ink discharge by the ink jet head, evaluation was carried out to see whether or not three-dimensional arrangement is possible, that is, whether or not cells can be arranged at arbitrary positions.

One hour after preparation of the "gel having cells arranged on the surface" described in each of the Examples and the Comparative Examples, the shape of the gel was observed under a confocal microscope (trade name, FV10; manufactured by Olympus Corporation) to see whether the cells arranged on the gel surface were immobilized near the surface. In cases where not less than half of the cells did not precipitate and were immobilized near the surface, the three-dimensional arrangement was rated as "good", while in cases where not less than half of the cells were present below half the height of the gel (including cases where the cells have precipitated to the bottom on the substrate), the three-dimensional arrangement was rated as "poor". The results are presented in Table 1 (see the "Three-dimensional arrangement" row in Table 1).

From the results in Table 1, it could be confirmed that the cells can be arbitrarily three-dimensionally arranged in cases where the concentrations of Tetra-PEG-maleimidyl and Tetra-PEG-SH are from 0.3% by mass to 20% by mass and, at the same time, the pHs of the buffers, that is, the pHs of Liquid A and Liquid B, are not less than 5. The alginate gel (Comparative Example 2) was also found to be capable of arbitrary three-dimensional arrangement of the cells. On the other hand, the fibrin gel (Comparative Example 4) was found to be incapable of three-dimensional arrangement of the cells at a concentration of 1.0% by mass, at which discharge by the ink jet head is possible. Similarly, Matrigel (Comparative Example 10) was found to be incapable of three-dimensional arrangement of cells at a concentration of 1.0% by mass, at which discharge by the ink jet head is possible.

### <Evaluation of Precision of Gel Shape Formation>

For each of Examples 1 to 34 and Comparative Examples 2 and 8, in which discharge of the liquid by the ink jet head was possible; the liquid was non-toxic to the cells; and three-dimensional arrangement of the cells was possible; the precision of gel shape formation was evaluated by calculating the aspect ratio of the dome-shaped gel formed.

For determination of the aspect ratio, the shape of the gel was observed under a confocal microscope, and then the diameter and the height of the dome-shaped gel were measured, followed by performing the following calculation: (gel height)/(gel diameter). In cases where the aspect ratio was not less than 0.19, which is the aspect ratio of an alginate gel conventionally used as a shape-forming agent (Comparative Example 2), the precision of shape formation was rated as "very good", while in cases where the aspect ratio was less than 0.19, the precision of shape formation was rated as "good". The results are presented in Table 1 (see the "Precision of shape formation" row in Table 1).

From the results in Table 1, it could be confirmed that, in cases where the concentrations of Tetra-PEG-maleimidyl and Tetra-PEG-SH are from 1.7% by mass to 20% by mass and, at the same time, the pHs of the buffers are not less than 6, the gel can have a higher aspect ratio than the aspect ratio of the alginate gel conventionally used as a shape-forming agent (Comparative Example 2), and hence can have an especially excellent precision of shape formation.

### <Evaluation of Shape Maintenance>

For each of Examples 1 to 34 and Comparative Examples 2 and 8, in which discharge of the liquid by the ink jet head was possible; the liquid was non-toxic to the cells; and three-dimensional arrangement of the cells was possible; evaluation of shape maintenance of the gel was carried out.

The preparation of the "gel having cells arranged on the surface" described in each of the Examples and the Comparative Examples was followed, 3 hours, 6 hours, 24 hours, 72 hours, or 168 hours later, by collection of 3 mL of the DMEM in the 3.5-cm dish into a 15-mL centrifuge tube using a micropipette. Instead, 3 mL of fresh DMEM supplemented with FBS was added to the dish, and the dish was then placed in the incubator (37°C, environment of 5% by volume CO₂) again. The single 15-mL centrifuge tube was subjected to centrifugation (trade name, H-19FM; manufactured by KOKUSAN Co., Ltd.; 1.2 × 10³ rpm, 5 minutes, 5°C), and then the supernatant was removed using an aspirator, followed by addition of 500 µL of fresh DMEM and pipetting, to obtain a cell suspension again.

The resulting cell suspension was placed in a well of a 96-well plate (trade name, 96-Well Cell Culture Plate; flat-bottomed; low evaporation type; provided with caps; polystyrene; manufactured by Corning) using a micropipette. Two hours after the addition to the well, the number of cells in the well was counted using a plate reader.

In cases where the total number of counted cells exceeded 750, that is, in cases where not less than one-quarter of all cells were detached from the gel surface, the shape maintenance was rated as "poor". In cases where the total number of counted cells did not exceed 750, that is, in cases where not less than three-quarters of all cells were maintained on the gel surface, the shape maintenance was rated as "good". The results are presented in Table 1 (see the "Shape maintenance" rows in Table 1).

From the results in Table 1, it could be confirmed that the shape of the gel can be maintained for not less than 168 hours in cases where the concentrations of Tetra-PEG-maleimidyl and Tetra-PEG-SH are from 0.3% by mass to 20% by mass and, at the same time, the pHs of the buffers, that is, the pHs of Liquid A and Liquid B, are from 5 to 10. On the other hand, in the case where the pHs of the buffers, that is, the pHs of Liquid A and Liquid B, were higher than 10 (Comparative Example 8), fragility of the gel caused shape degradation in 6 hours after the shape formation. Further, the alginate gel (Comparative Example 2) was also found to undergo shape degradation in 6 hours after the shape formation.

### <Evaluation of Cell Morphology on Three-Dimensional Structure>

For each of Examples 1 to 34, in which discharge of the liquid by the ink jet head was possible; the liquid was non-toxic to the cells; three-dimensional arrangement of the cells was possible; and the shape could be maintained; evaluation of the morphology of the cells arranged on the surface of the three-dimensional structure, that is, the "gel having cells arranged on the surface", was carried out after 24 hours, 72 hours, or 168 hours of culture. Further, for reference, Comparative Example 2, in which not less than one-quarter of all cells were detached from the gel surface due to failure in maintenance of the shape in the evaluation of shape maintenance, was similarly subjected to the evaluation using only a sample after 24 hours of culture. The evaluation was not carried out for a sample after 72 hours of culture and a sample after 168 hours of culture because of collapse of the three-dimensional structure.

The morphology of the cells arranged on the three-dimensional structure, that is, the "gel having cells arranged on the surface", was observed in order to evaluate whether spreading of the cells occurred or not. The evaluation results are presented in Table 1 (see the "Cell morphology 1" rows in Table 1). In cases where almost all arranged cells exhibited spreading, the cell morphology was rated as A. In cases where the arranged cells did not exhibit spreading, the cell morphology was rated as C. Whether or not the spreading of cells occurred was judged based on finding of pseudopods of the cells.

From the results in Table 1, it could be confirmed that spreading of the cells on the three-dimensional structure occurs at least in cases where from 0.1 to 1.5% by mass fibrinogen is added, or at least in cases where from 0.05 to 1.0% by mass Matrigel is added as a self-assembling biomaterial.

### <Evaluation of Survival Rate in Three-Dimensional Layers>

For each of Examples 1 to 34, in which discharge of the liquid by the ink jet head was possible; the liquid was non-toxic to the cells; three-dimensional arrangement of the cells was possible; and the shape could be maintained; whether or not the cells can survive in the "gel containing three-dimensionally layered cells therein" was evaluated. Further, for reference, Comparative Example 2, in which not less than one-quarter of all cells were detached from the gel surface due to failure in maintenance of the shape in the evaluation of shape maintenance, was similarly subjected to the evaluation. Comparative Example 3, in which discharge by the ink jet head was impossible, was also similarly subjected to the evaluation using a manually prepared "gel containing three-dimensionally layered cells therein". The survival rate of the cells in the gel was calculated by LIVE/DEAD staining without destroying the three-dimensional layers.

### • Preparation of Culture Medium for LIVE/DEAD Staining

To 60 mL of DMEM supplemented with FBS, 30 µL of PI solution and 12 µL of Hoechst 33342 (Product No. H3570; manufactured by Life Technologies) were added, to prepare a culture medium for LIVE/DEAD staining.

### • Capture of Image for Calculation of Survival Rate

The "gel containing three-dimensionally arranged cells therein" in each of Examples 1 to 34, Comparative Example 2, and Comparative Example 3 was cultured for 24 hours, and then 3 mL of the DMEM supplemented with FBS in the 3.5-cm dish was replaced with 3 mL of the culture medium for evaluation of the survival rate, followed by culturing the cells again for 1 hour in an incubator (37°C, environment of 5% by volume CO₂). Thereafter, the cells in the gel were observed under a confocal microscope, and a three-dimensional image of the cells was saved as a TIFF file. Comparative Example 2 and Comparative Example 3 showed detachment of the gel from the substrate during the 24 hours of culture, and the size of the gel was found to have been reduced to about half relative to the size found immediately after its preparation. Nevertheless, the survival rate was similarly calculated.

For Examples 2 to 4, 7 to 19, and 21 to 34, evaluation was carried out also after additional 48 hours of culture (that is, after a total of 72 hours of culture) similarly to the evaluation after the 24 hours of culture, to calculate the survival rate.

For Examples 2 to 4, 7 and 8, 11 and 12, 16 to 19, and 21 to 34, evaluation was carried out also after additional 96 hours of culture (that is, after a total of 168 hours of culture) similarly to the evaluation after the 24 hours of culture, to calculate the survival rate.

### • Calculation of Survival Rate by LIVE/DEAD Staining

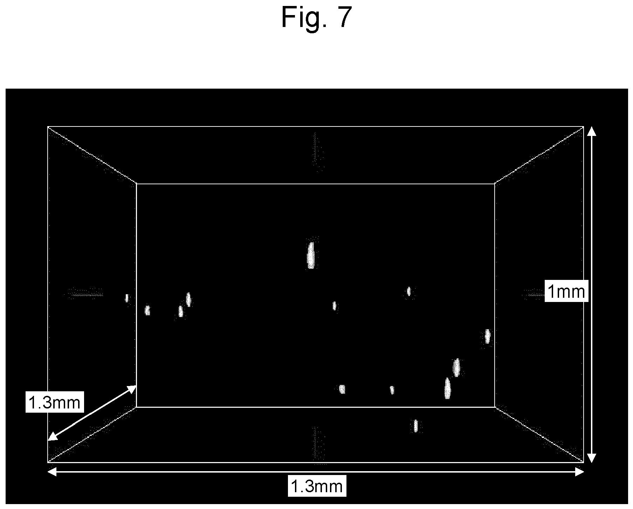

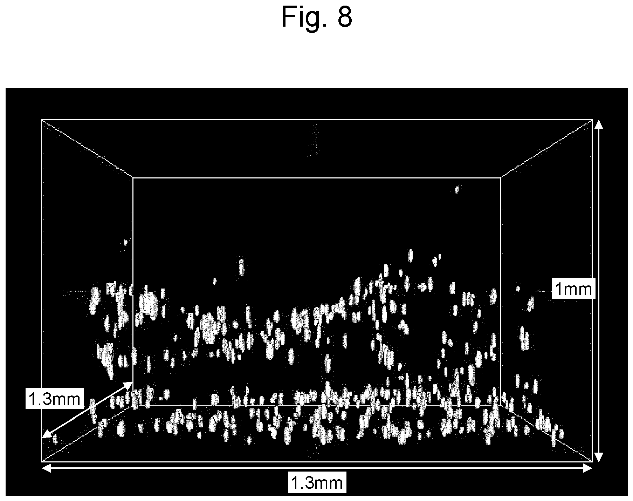

The TIFF file was converted to the stack format using image processing software MetaMorph (manufactured by Molecular Devices Japan Co., Ltd.), and separated on a color basis using image processing software ImageJ. Each of the red and blue images was subjected to binarization, and then to noise reduction and a process for separation of aggregated cells. Each processed image was subjected to z-axis correction using MetaMorph again, and then the cell number was counted. Cells stained with PI solution were regarded as dead cells, and cells stained with Hoechst 33342 were regarded as total cells. The survival rate (%) was calculated as follows: 100 - (number of dead cells) × 100/(total number of cells). FIG. 7 is an image of dead cells in Example 13 as viewed using 4D Viewer. FIG. 8 is an image of all cells in Example 13 as viewed using 4D Viewer.

### • Evaluation Standard

In cases where the cell survival rate in the three-dimensional layers was not less than 90%, the survival rate was rated as "very good". In cases where the cell survival rate was not less than 75% and less than 90%, the survival rate was rated as "good". In cases where the cell survival rate was less than 75%, the survival rate was rated as "poor". The results are presented in Table 1 (see the "Survival rate" rows in Table 1).

From the results in Table 1, it could be confirmed that the cells can survive in the three-dimensional layers under conditions where discharge of the liquid by the ink jet head was possible; the liquid was non-toxic to the cells; three-dimensional arrangement of the cells was possible; and the shape could be maintained; which conditions correspond to the cases where the concentrations of Tetra-PEG-maleimidyl and Tetra-PEG-SH were from 0.3% by mass to 20% by mass and, at the same time, the pHs of the buffers, that is, the pHs of Liquid A and Liquid B, were from 5 to 10.

Further, it could be confirmed that the cells can survive for not less than 72 hours in the three-dimensional layers at least in cases where the concentrations of Tetra-PEG-maleimidyl and Tetra-PEG-SH are from 0.3% by mass to 6.0% by mass.

Further, it could be confirmed that the cells can survive for not less than 168 hours in the three-dimensional layers at least in cases where the concentrations of Tetra-PEG-maleimidyl and Tetra-PEG-SH are from 0.3% by mass to 4.0% by mass.

On the other hand, it could be confirmed that cells cannot survive in the three-dimensional layers of alginate gel (Comparative Example 2 and Comparative Example 3).

### <Evaluation of Cell Morphology in Three-Dimensional Layers>

For each of Examples 1 to 34, in which discharge of the liquid by the ink jet head was possible; the liquid was non-toxic to the cells; three-dimensional arrangement of the cells was possible; the shape could be maintained; and the cells could survive in the three-dimensional layers; the cell morphology in the "gel containing three-dimensionally layered cells therein" after 24 hours of culture was evaluated.

For Examples 2 to 4, 7 to 19, and 21 to 34, evaluation was carried out also after additional 48 hours of culture (that is, after a total of 72 hours of culture) similarly to the evaluation after the 24 hours of culture, to evaluate the cell morphology.

For Examples 2 to 4, 7 and 8, 11 and 12, 16 to 19, and 21 to 34, evaluation was carried out also after additional 96 hours of culture (that is, after a total of 168 hours of culture) similarly to the evaluation after the 24 hours of culture, to evaluate the cell morphology.

Further, for reference, Comparative Example 2, in which the cells could not survive in the three-dimensional layers, was similarly subjected to the evaluation for only a sample after 24 hours of culture. Comparative Example 3 was also similarly subjected to the evaluation for only a sample after 24 hours of culture, using a manually prepared "gel containing three-dimensionally layered cells therein". The evaluation was not carried out for a sample after 72 hours of culture and a sample after 168 hours of culture because of collapse of the three-dimensional structure.

### • Preparation of Culture Medium for Evaluation of Cell Morphology

To 60 mL of DMEM supplemented with FBS, 12 µL of AM (Product No. L3224, manufactured by Life Technologies) was added, to prepare a culture medium for evaluation of the cell morphology.

### • Evaluation of Cell Morphology

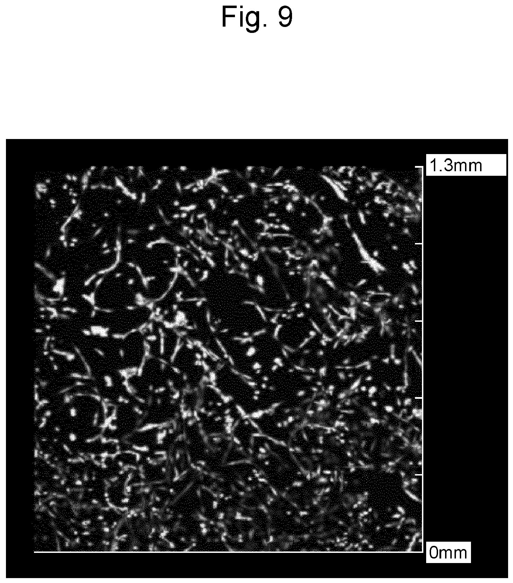

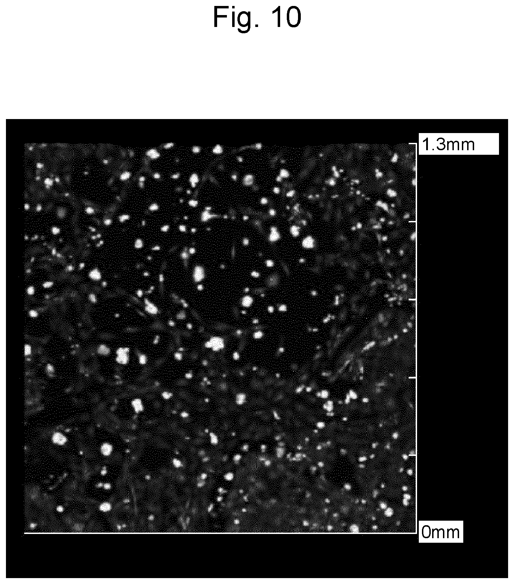

After the calculation of the survival rate in the evaluation of the survival rate in the three-dimensional layers, 3 mL of the culture medium for the calculation of the survival rate in the 3.5-cm dish was replaced with 3 mL of the culture medium for evaluation of the cell morphology. Culture was carried out again for 1 hour in an incubator (37°C, environment of 5% by volume CO₂). Thereafter, the morphology of the cells in the three-dimensional layers was observed under a confocal microscope. FIG. 9 and FIG. 10 are images obtained by observation, from the upper side, of the cells in the three-dimensional layers in Examples 13 and 15, respectively.

### • Evaluation Standard

In cases where almost all cells exhibited spreading, the cell morphology was rated as A. In cases where not less than half of the cells exhibited spreading, the cell morphology was rated as B. In cases where the cells did not exhibit spreading, the cell morphology was rated as C. Whether or not the spreading occurred was judged based on finding of pseudopods of the cells. The results are presented in Table 1 (see the "Cell morphology 2" rows in Table 1).

From the results in Table 1, it could be confirmed that spreading of the cells in the three-dimensional layers occurs at least in cases where from 0.1 to 1.5% by mass fibrinogen is added, or at least in cases where from 0.05 to 1.0% by mass Matrigel is added as a self-assembling biomaterial. Further, it could be confirmed that spreading of almost all cells in the three-dimensional layers occurs in cases where the concentrations of Tetra-PEG-maleimidyl and Tetra-PEG-SH are from 0.3% by mass to 6.0% by mass.

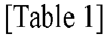

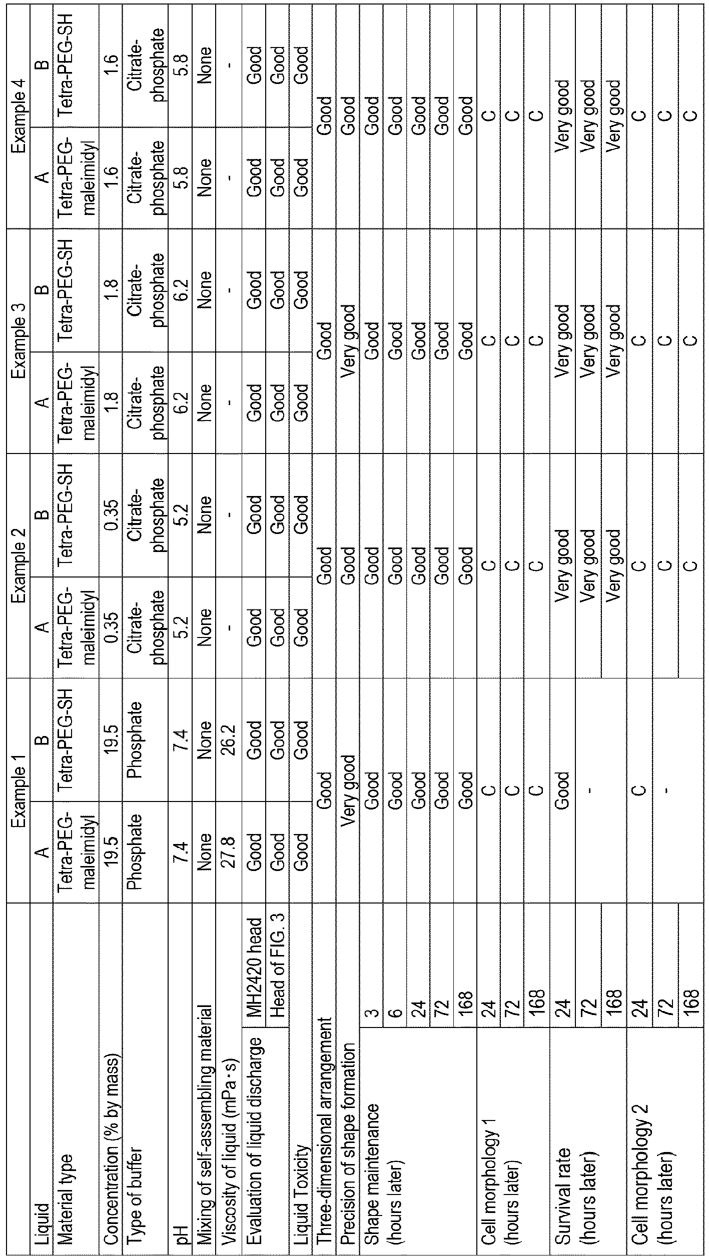

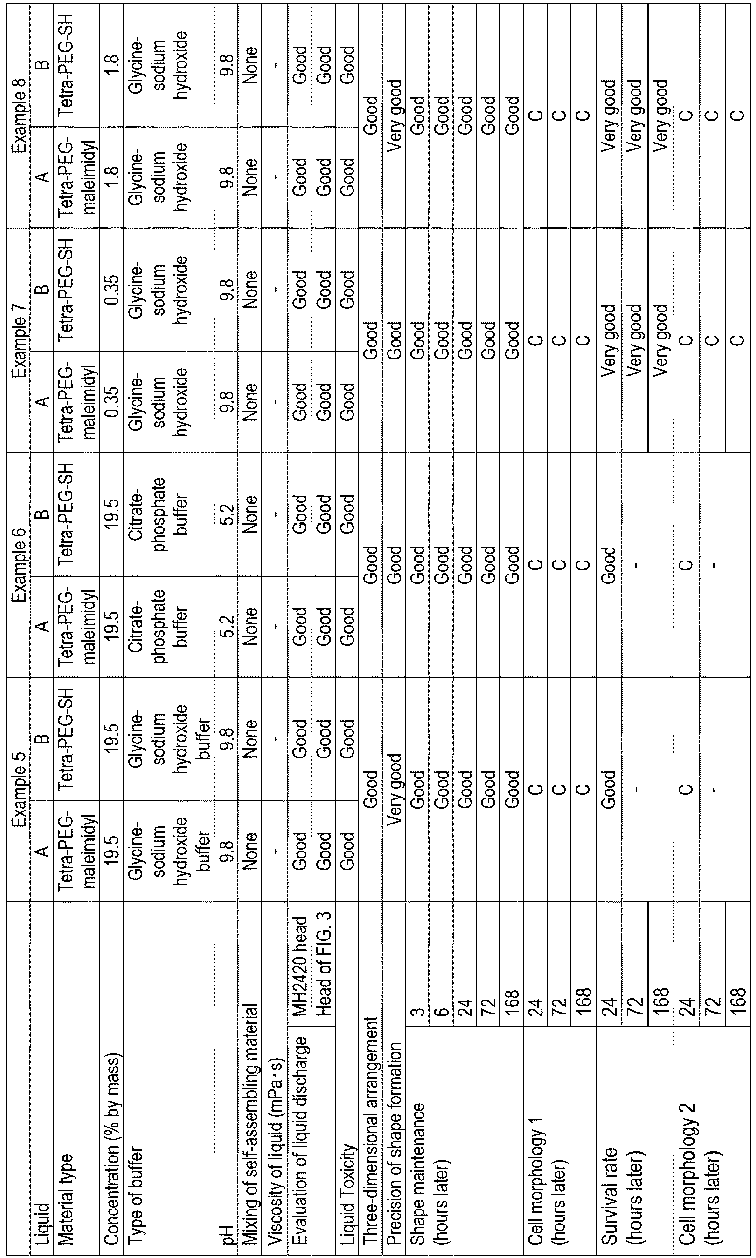

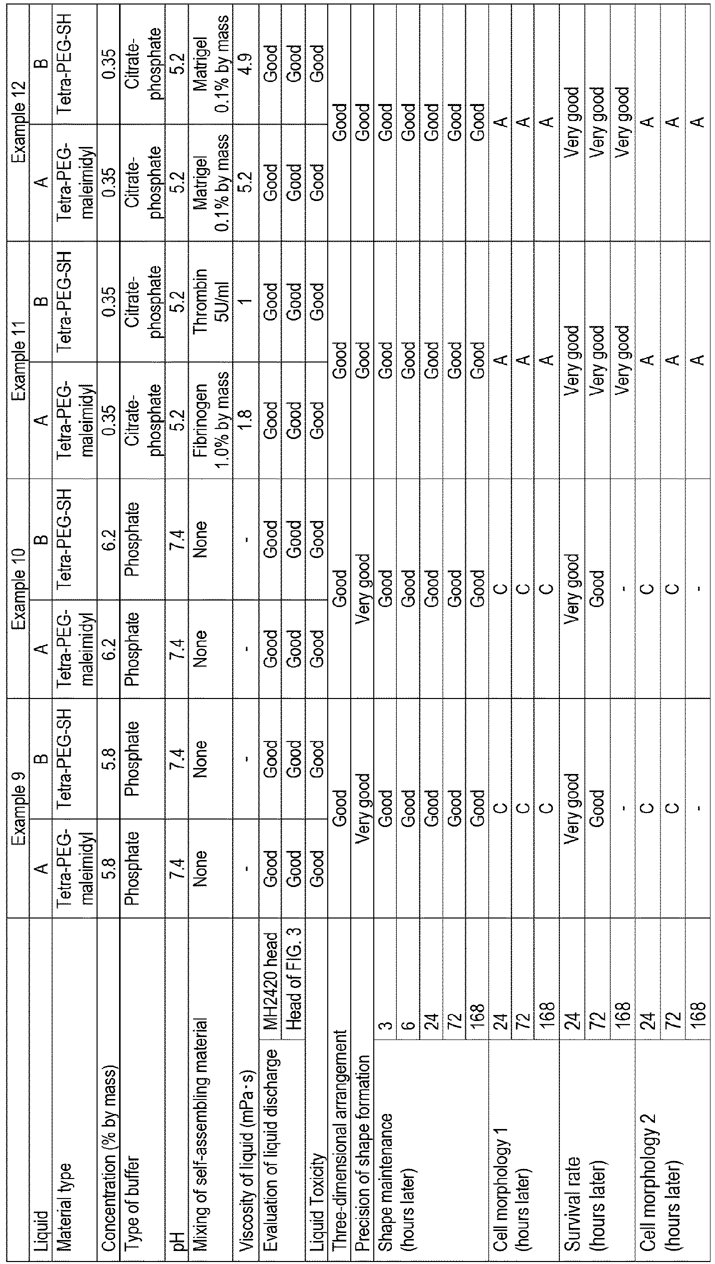

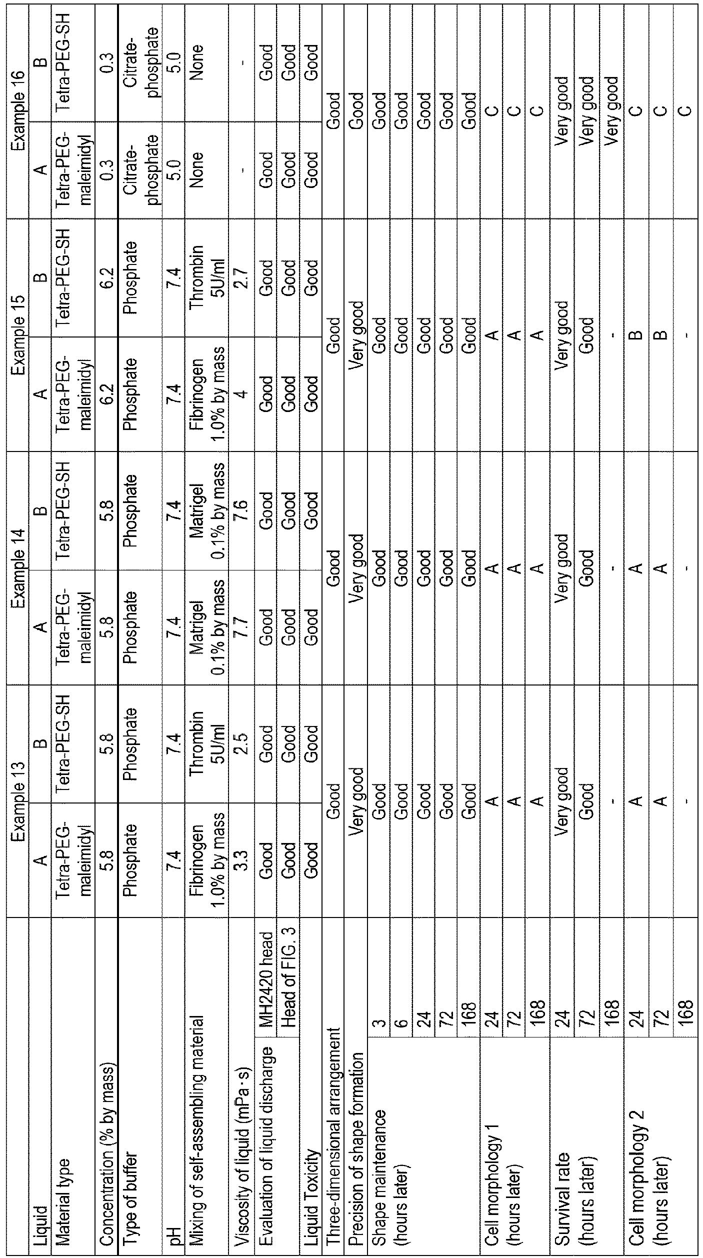

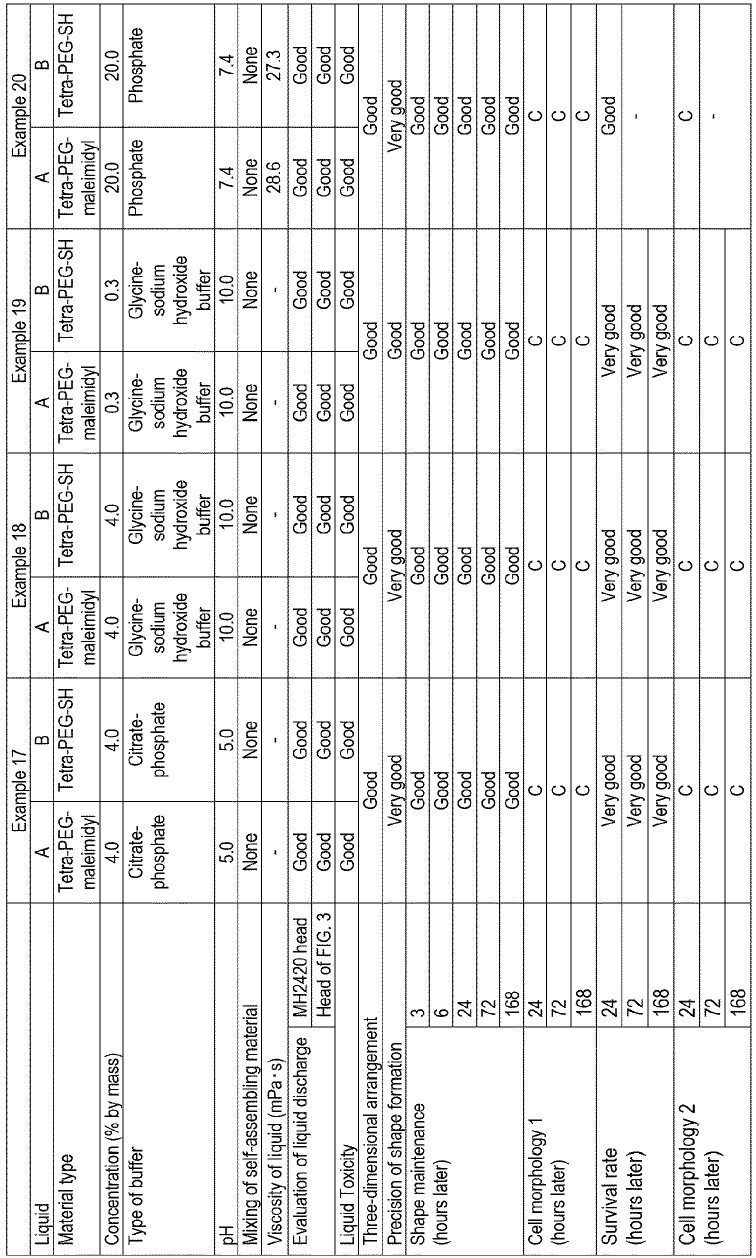

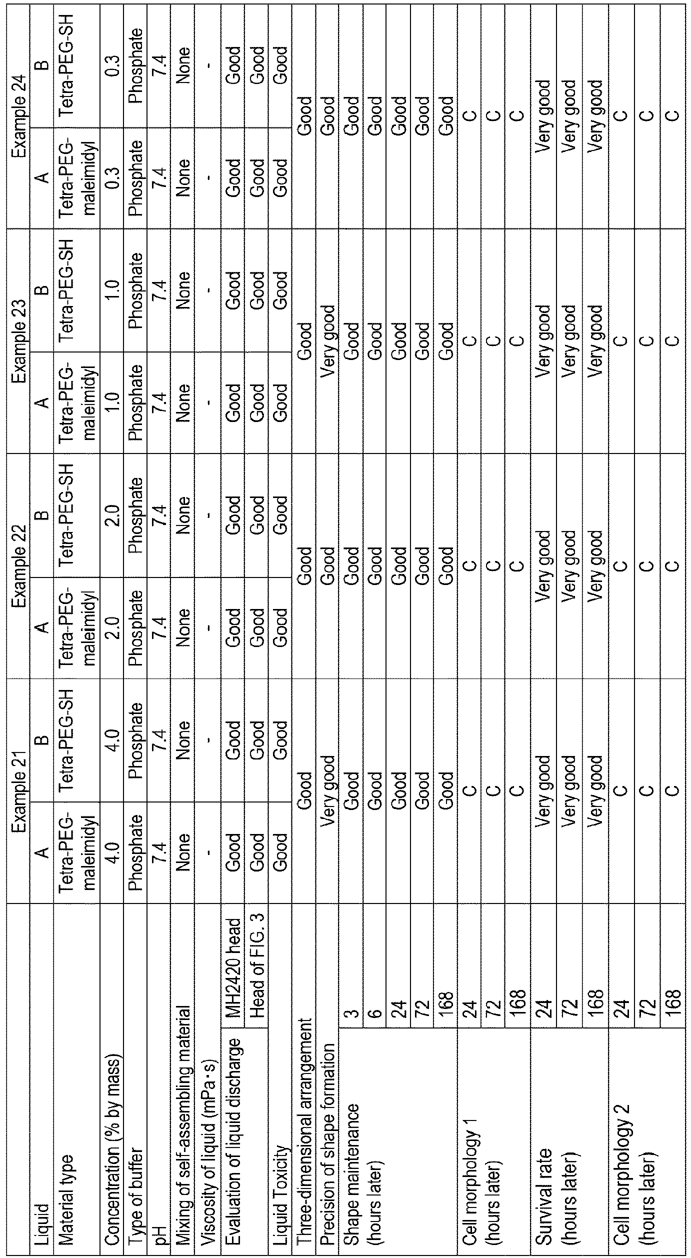

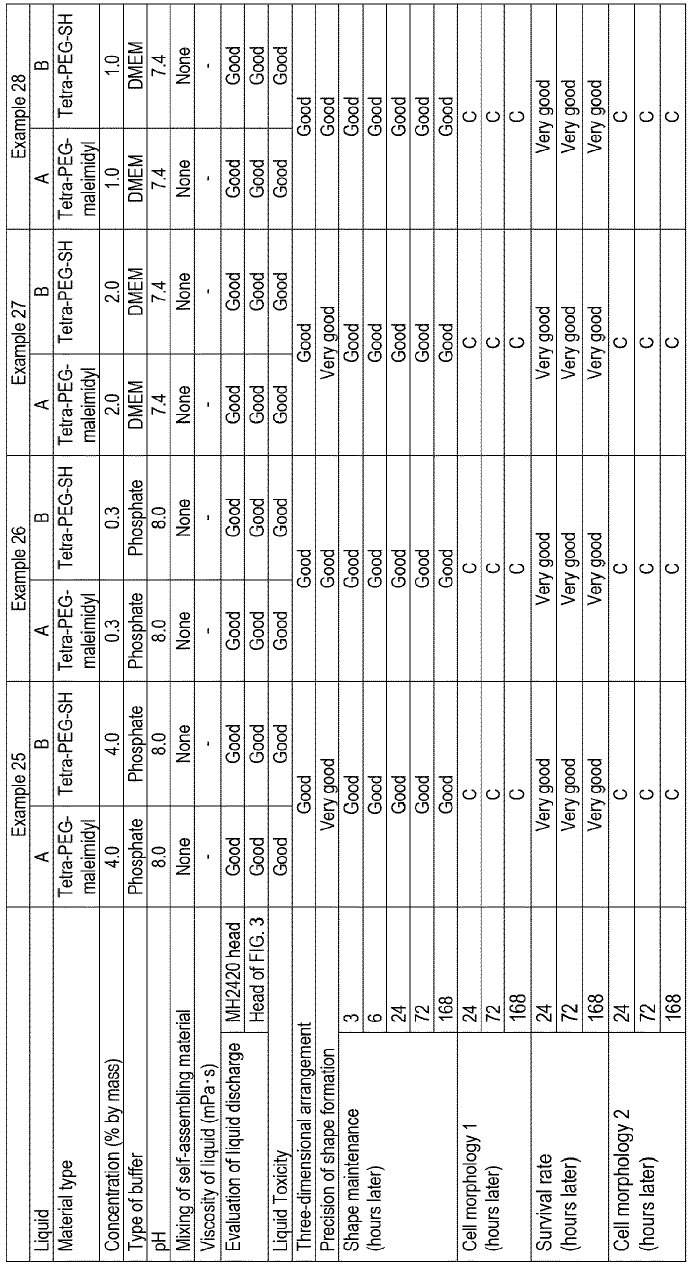

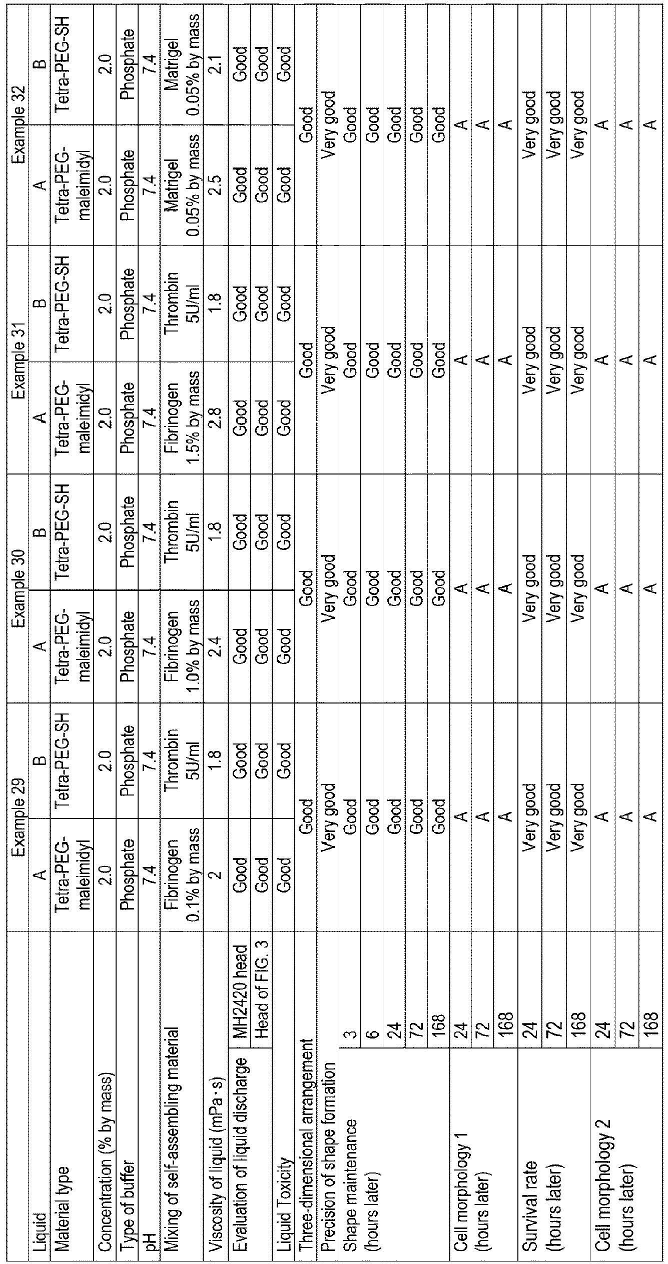

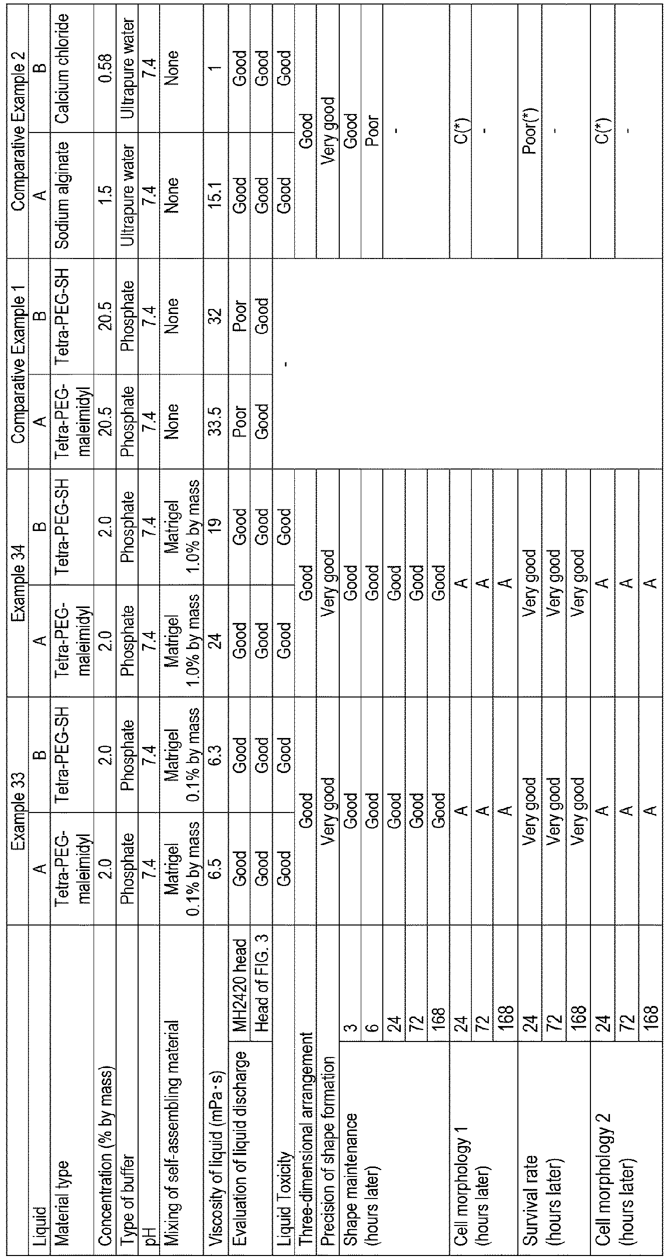

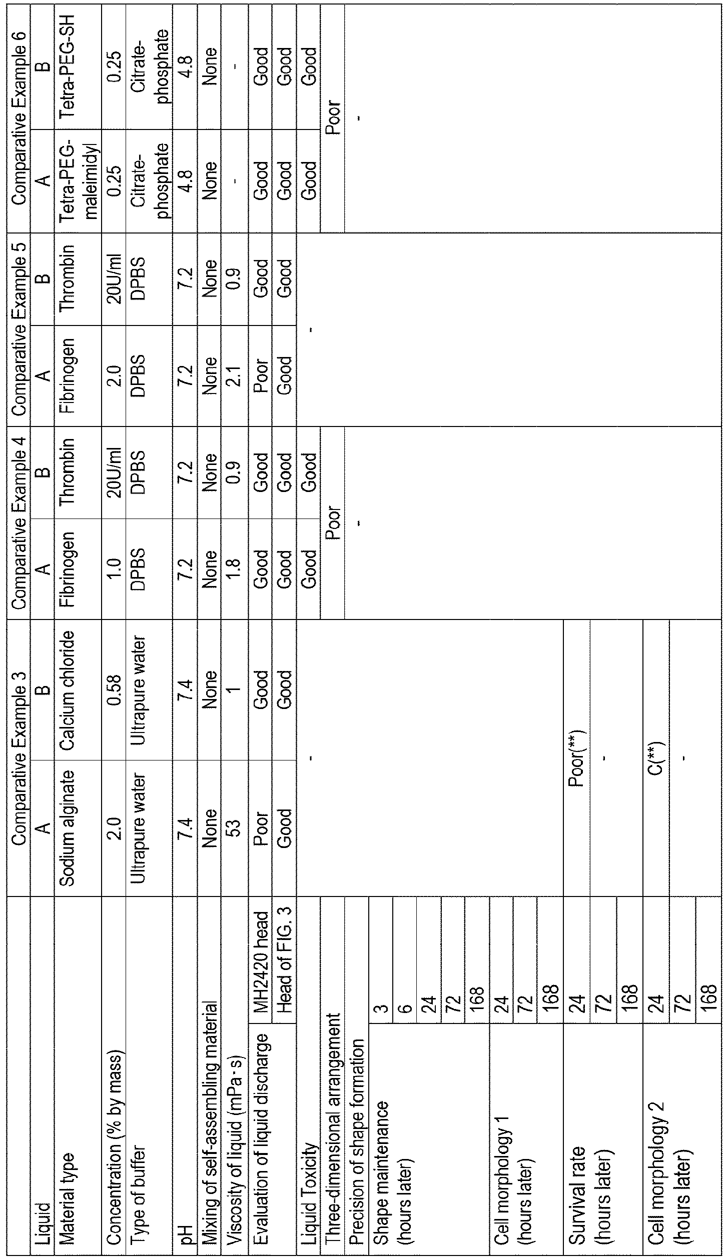

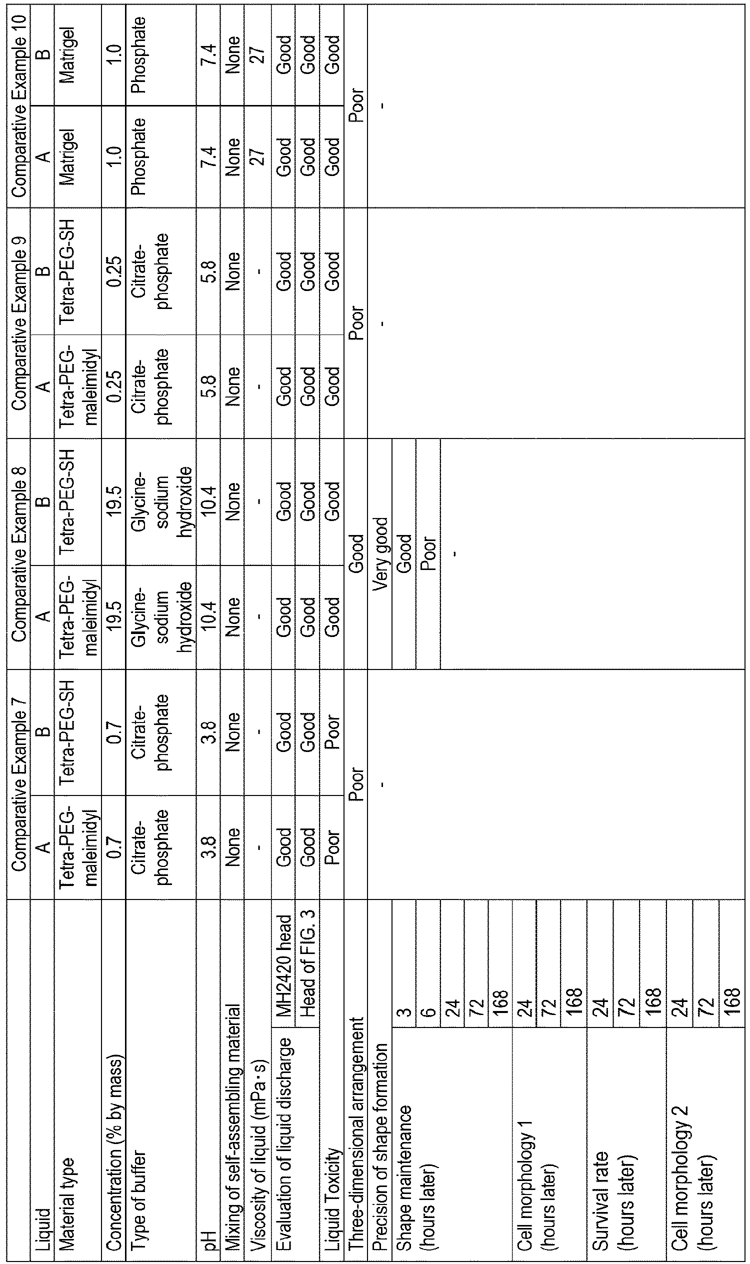

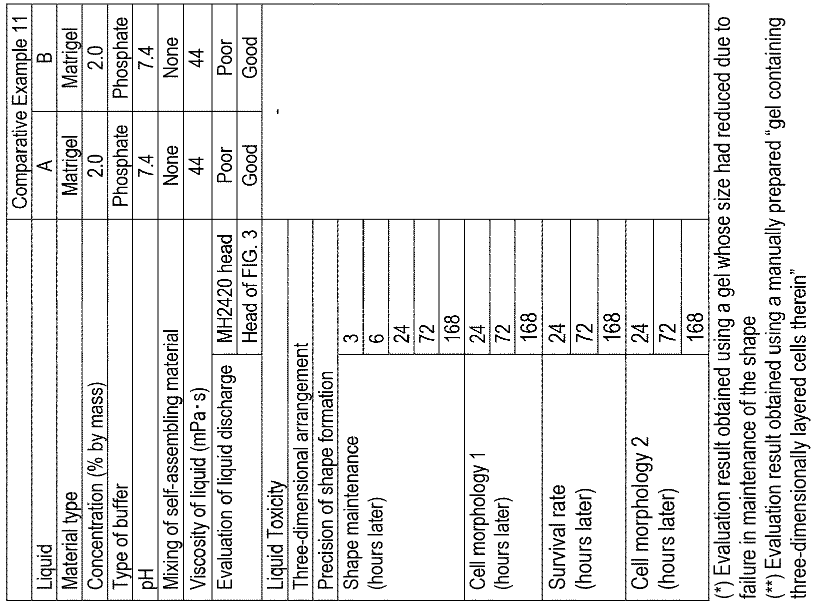

From the results of the measurement of the viscosity of the liquid, the evaluation of discharge of the liquid by the ink jet head, the evaluation of toxicity of the liquid, the evaluation of three-dimensional arrangement of the cells, the evaluation of maintenance of the shape, and the evaluation of the survival rate in the three-dimensional layers in Table 1, it could be confirmed that, in each case where the concentrations of Tetra-PEG-maleimidyl and Tetra-PEG-SH are from 0.3% by mass to 20% by mass (the viscosities of Liquid A and Liquid B at 25°C are not more than 30 mPa·s), and at the same time, the pHs of the buffers, that is, the pHs of Liquid A and Liquid B, are from 5 to 10, discharge of the liquid by the ink jet head is possible; the liquid is non-toxic to the cells; the cells can be arbitrarily three-dimensionally arranged; the shape can be maintained; and the cells can survive in the three-dimensional layers.

Further, from the results of the evaluation of the survival rate in the three-dimensional layers in Table 1, it could be confirmed that, in cases where the concentrations of Tetra-PEG-maleimidyl and Tetra-PEG-SH are from 0.3% by mass to 4.0% by mass, the cells can maintain a high survival rate even after not less than 168 hours of long-term culture.

From the results of the evaluation of the precision of shape formation in Table 1, it could be confirmed that an especially excellent precision of shape formation can be achieved in cases where the concentrations of Tetra-PEG-maleimidyl and Tetra-PEG-SH are from 1.7% by mass to 20% by mass and, at the same time, the pHs of the buffers, that is, the pHs of Liquid A and Liquid B, are from 6 to 10.

Further, from the results of the evaluation of the cell morphology on the three-dimensional structure and the evaluation of the cell morphology in the three-dimensional layers in Table 1, it could be confirmed that the cells are capable of spreading on the three-dimensional structure or in the three-dimensional layers in cases where Liquid A and Liquid B contain a self-assembling biomaterial. Further, it could be confirmed that almost all cells in the three-dimensional layers exhibit spreading in cases where the concentrations of Tetra-PEG-maleimidyl and Tetra-PEG-SH are from 0.3% by mass to 6.0% by mass.

Examples of modes of the present invention include the following <1> to <11>.
<1> A liquid set for a droplet discharging apparatus, the liquid set including:
   Liquid A containing a multi-branched polymer A, the polymer containing, as a backbone, a polyethylene glycol containing at least three branches, the branches containing one or more electrophilic functional groups in at least one of a side chain(s) and an end(s); and
   Liquid B containing a multi-branched polymer B, the polymer containing, as a backbone, a polyethylene glycol containing at least three branches, the branches containing one or more nucleophilic functional groups in at least one of a side chain(s) and an end(s);
   the Liquid A and the Liquid B each having a pH of from 5 to 10, and containing the multi-branched polymer at a concentration of from 0.3% by mass to 20% by mass.
<2> The liquid set for a droplet discharging apparatus according to <1>, wherein the electrophilic functional group is selected from the group consisting of maleimidyl, N-hydroxy-succinimidyl (NHS), sulfosuccinimidyl, phthalimidyl, imidazoyl, acryloyl, and nitrophenyl, and the nucleophilic functional group is selected from the group consisting of thiol, amino, and -CO₂PhNO₂.
<3> The liquid set for a droplet discharging apparatus according to <1> or <2>, wherein both the multi-branched polymer A and the multi-branched polymer B are four-branched polymers.
<4> The liquid set for a droplet discharging apparatus according to any one of <1> to <3>, wherein the electrophilic functional group is maleimidyl, and the nucleophilic functional group is thiol.
<5> The liquid set for a droplet discharging apparatus according to any one of <1> to <4>, wherein each of the Liquid A and the Liquid B has a viscosity of not more than 30 mPa·s at 25°C.
<6> The liquid set for a droplet discharging apparatus according to any one of <1> to <5>, wherein the multi-branched polymer in each of the Liquid A and the Liquid B has a concentration of from 0.3% by mass to 6.0% by mass.
<7> The liquid set for a droplet discharging apparatus according to any one of <1> to <6>, wherein the multi-branched polymer in each of the Liquid A and the Liquid B has a concentration of from 0.3% by mass to 4.0% by mass.
<8> The liquid set for a droplet discharging apparatus according to any one of <1> to <5>, wherein each of the Liquid A and the Liquid B has a pH of from 6 to 10, and the multi-branched polymer in each of the Liquid A and the Liquid B has a concentration of from 1.7% by mass to 20% by mass.
<9> The liquid set for a droplet discharging apparatus according to any one of <1> to <8>, wherein one or both of the Liquid A and the Liquid B contains a self-assembling biomaterial.
<10> The liquid set for a droplet discharging apparatus according to <9>, wherein the self-assembling biomaterial is one or more selected from the group consisting of: a gel containing laminin and collagen; fibrinogen; gelatin; and elastin.
<11> The liquid set for a droplet discharging apparatus according to any one of <1> to <10>, wherein one or both of the Liquid A and the Liquid B contains suspended cells.

With the liquid set for a droplet discharging apparatus according to any one of <1> to <11>, the conventional problems can be solved to achieve the object of the present invention.

## Claims

1. A liquid set for a droplet discharging apparatus, the liquid set comprising:
Liquid A containing a multi-branched polymer A, the polymer containing, as a backbone, a polyethylene glycol containing at least three branches, the branches containing one or more electrophilic functional groups in at least one of a side chain(s) and an end(s); and
Liquid B containing a multi-branched polymer B, the polymer containing, as a backbone, a polyethylene glycol containing at least three branches, the branches containing one or more nucleophilic functional groups in at least one of a side chain(s) and an end(s);
the Liquid A and the Liquid B each having a pH of from 5 to 10, and containing the multi-branched polymer at a concentration of from 0.3% by mass to 20% by mass.

2. The liquid set for a droplet discharging apparatus according to claim 1, wherein the electrophilic functional group is selected from the group consisting of maleimidyl, N-hydroxy-succinimidyl (NHS), sulfosuccinimidyl, phthalimidyl, imidazoyl, acryloyl, and nitrophenyl, and the nucleophilic functional group is selected from the group consisting of thiol, amino, and -CO₂PhNO₂.

3. The liquid set for a droplet discharging apparatus according to claim 1 or 2, wherein both the multi-branched polymer A and the multi-branched polymer B are four-branched polymers.

4. The liquid set for a droplet discharging apparatus according to any one of claims 1 to 3, wherein the electrophilic functional group is maleimidyl, and the nucleophilic functional group is thiol.

5. The liquid set for a droplet discharging apparatus according to any one of claims 1 to 4, wherein each of the Liquid A and the Liquid B has a viscosity of not more than 30 mPa·s at 25°C.

6. The liquid set for a droplet discharging apparatus according to any one of claims 1 to 5, wherein the multi-branched polymer in each of the Liquid A and the Liquid B has a concentration of from 0.3% by mass to 6.0% by mass.

7. The liquid set for a droplet discharging apparatus according to any one of claims 1 to 6, wherein the multi-branched polymer in each of the Liquid A and the Liquid B has a concentration of from 0.3% by mass to 4.0% by mass.

8. The liquid set for a droplet discharging apparatus according to any one of claims 1 to 5, wherein each of the Liquid A and the Liquid B has a pH of from 6 to 10, and the multi-branched polymer in each of the Liquid A and the Liquid B has a concentration of from 1.7% by mass to 20% by mass.

9. The liquid set for a droplet discharging apparatus according to any one of claims 1 to 8, wherein one or both of the Liquid A and the Liquid B contains a self-assembling biomaterial.

10. The liquid set for a droplet discharging apparatus according to claim 9, wherein the self-assembling biomaterial is one or more selected from the group consisting of: a gel containing laminin and collagen; fibrinogen; gelatin; and elastin.

11. The liquid set for a droplet discharging apparatus according to any one of claims 1 to 10, wherein one or both of the Liquid A and the Liquid B contains suspended cells.
